# EUROPEAN PATENT SPECIFICATION

(11) **EP 3 403 663 B1**
(45) Date of publication and mention of the grant of the patent: **27.08.2025**
(21) Application number: 17201472.2
(22) Date of filing: 15.05.2012
(51) Int. Cl.: A61K 36/29, A61K 36/48, A61K 36/23, A61K 36/899, A61K 36/77, A61K 36/28, A61K 36/76, A61K 36/35, A61K 36/22, A61K 36/27, A61K 36/31, A61K 36/808, A61K 36/90, A61K 36/66, A61K 36/68, A61P 1/00, A61P 9/14, A61P 11/00, A61P 29/00, A61P 43/00

(54) **THERAPEUTIC COMPOSITIONS OF SPECIFIED HERBAL FORMULATIONS AND USES THEREOF**
THERAPEUTISCHE ZUSAMMENSETZUNGEN AUS BESTIMMTEN KRÄUTERFORMULIERUNGEN UND VERWENDUNGEN DAVON
COMPOSITIONS THÉRAPEUTIQUES DE FORMULATIONS À BASE D'HERBES SPÉCIFIÉES ET LEURS UTILISATIONS

(30) Priority: 16.05.2011 US 201161486724 P
(43) Date of publication of application: 21.11.2018
(62) Divisional of application: 12865478.7
(73) Proprietor: Haus Bioceuticals, Inc., Oklahoma City, OK 73104 (US)
(72) Inventor: YESUDAS, Tomy, Kollam 691503 Kerala (IN); ALEX, Philip, Radnor, PA 19087 (US); CENTOLA, Michael, Oklahoma City, OK Oklahoma 73104 (US); PAYNE, Adam, Joshua, Edmond, OK Oklahoma 73034 (US)
(74) Representative: Schiweck Weinzierl Koch Patentanwälte Partnerschaft mbB

(56) References cited:
- No Search

## Description

### RELATED APPLICATIONS

This application claims priority to U.S. Provisional Application No. 61/486,724, filed May 16, 2011, by Payne et al., and entitled "THERAPEUTIC COMPOSITIONS OF SPECIFIED HERBAL FORMULATIONS AND USES THEREOF".

### FIELD OF THE INVENTION

The present invention is in the field of plant formulations used as pharmaceutical compositions plant for the management of skin conditions and methods of using the same.

### BACKGROUND OF THE DISCLOSURE

Herbs have been used in the practice of medicine for thousands of years. Therapeutic uses of plant extracts have evolved over millennia. Plant derived therapies such as Penicillin and Taxol revolutionized the fight against infection and breast cancer respectively, demonstrating that the knowledge obtained through simple trial and error, evidenced from their continued use represents a valuable resource that may lead to novel treatments, or new insights into pathogenesis. Driven by increasing public interest, health care practitioners and the medical research community have begun to demonstrate great interest in the therapeutic uses of plant medicines, which represent a rapidly growing area of public interest (over 38 million consumers in the LTS alone in 2005). However, for the majority of plant medicines, issues pertaining to their inadequate efficacy studies and lack of information on mechanism of action (MOA) remain, significantly limiting their use as effective medical alternatives. The lack of well-defined molecular mechanisms of plant medicines leads to frustration for both healthcare professionals and the public when decisions are to be made on their use as potentially effective medical alternatives or options, particularly in chronic inflammatory conditions, where limited safe therapeutic options are available.

Wolf et al. described a comparison of a Hamamelis preparation with dexpanthenol for use at children with skin disorders and found that Hamamelis ointment is a safe and effective treatment of skin orders of children up to the age of 11 years (cf. ref. 8).

Abu Bakr Mohammad.Bin Zakariyya Al-Razi; Kitaab-al-Haawi-fi1-Tibb, Vol.XI (9th century AD), Dayerah-al- Ma'aarif Usmani a, Hyderabad, (First Edition) 1962 AD Pg 202, Formulation ID: AA11/750A, Formulation Name: Natool Bara-e-Niqras; found in the Traditional Knowledge Digital Library (TKDL), disclose the use of a preparation containing Colchicum autumnale, Populus alba, and Achillea millefolium for the treatment of gout.

### SUMMARY OF THE INVENTION

Disclosed herein is a composition comprising a mixture of plant extracts comprising:
*an extract of Althaea officinalis, an extract of Conium maculatum, an extract of Ervum lens, an extract of Phytolacca decandra, an extract of Populus alba, an extract of Populus tremuloides, an extract of Sambucus nigra, an extract of Hamamelis virginiana, an extract of Pimpinella saxifraga, an extract of Rhus toxicodendron, an extract of Vincetoxicum officinale, an extract of Achillea millefolium, an extract of Avena sativa, an extract of Sanguinaria canadensis, an extract of Berberis vulgaris, an extract of Cochlearia officinalis, an extract of Hydrastis canadensis, an extract of Matricaria chamomilla, an extract of Nasturtium officinale, an extract of Scrophularia nodosa, an extract of Smilax medica, an extract of Tussilago farfara, and an extract of Veronica officinalis.*Also disclosed are pharmaceutical compositions comprising the above composition and a pharmaceutically acceptable carrier, diluents, or excipient. In addition, disclosed herein is the above composition for use in reducing symptoms associated with a skin condition in a subject.

### BRIEF DESCRIPTION OF THE DRAWINGS

Figure 1 is a representation of the immunomodulatory effects exerted by HAT-01 through its regulation of TLR functions on monocytes and keratinocytes.
Figure 2 demonstrates the therapeutic benefit of HAT-01 through its anti-inflammatory effects suppressing mice from developing systemic inflammation in murine models of colitis.
Figure 3 is a depiction of the immunomodulatory and anti-inflammatory role of HAT-01 in a murine model of colitis modulated by distinct immunosuppressive cytokine pattern and mediated by NFκB & MAPK.
Figure 4 is a representation of the effects of HAT-01 to significantly suppress mice from developing endotoxemia.
Figure 5 is a demonstration of the novel clinical therapeutic effects of HAT-01 in a murine model of chronic atopic dermatitis.
Figure 6 is a demonstration of the immunomodulatory and immunosuppressive effects of HAT-01 in murine chronic atopic dermatitis.
Figure 7 is an illustration of the pilot trial study design conducted to evaluate the safety, efficacy & tolerability of HAT-01 in patients with atopic dermatitis.
Figure 8 demonstrates the clinical and therapeutic benefit of HAT-01 in a pilot clinical trial study of patients with atopic dermatitis.
Figure 9 is a demonstration of the therapeutic benefits of HAT-01 in a pilot trial study of atopic dermatitis as measured by peripheral blood eosinophil counts.
Figure 10 illustrates the clinical efficacy of HAT-01 in a randomized placebo-controlled clinical trial in patients with atopic dermatitis.
Figure 11 is a comparative analysis of the therapeutic benefits of HAT-01 in murine and human models of atopic dermatitis.
Figure 12 is a graph showing the results of the application of the HAT-01 formulation to psoriasis patients.
Figure 13 illustrates the efficacy of HAT-01 as compared with other common treatments for atopic dermatitis.

### DETAILED DESCRIPTION OF THE EMBODIMENTS

Disclosed herein are plant compositions, based on a proprietary preparation of traditional commonly used herbs that have long been used to treat inflammatory conditions. Our studies have demonstrated that this formulation is a potent, safe, anti-inflammatory agent with therapeutic benefit in the chronic inflammatory conditions tested as described herein (atopic dermatitis, inflammatory bowel disease and experimental sepsis).

Thus, in the first aspect, disclosed herein is complex plant preparation containing a unique mixture of traditional commonly used multiple plants/herbs. In certain embodiments, the disclosed preparations comprise plant extracts, which have long been used to treat inflammatory conditions.

The term "plant extract" or "plant extracts" as used herein refers to a composition of extracted plant substances, including but not limited to: juices; macerations; concentrates; syrups; cold, heated, fluid and/or fermented extracts; powders; tablets; tinctures; herbal teas in the form of infusions or decoctions; poultices; lotions; creams; gels; ointments; salves; liniments; balms; oils; essential oils; suspensions; emulsions; compresses; dressings; fomentations; eyebaths; gargles; enemas; boluses; and other forms of extracted plant substances such as are known to those of ordinary skill in the arts of pharmacognosy, herbalism, and medicinal formulation. As used herein the term "plant extract" or "plant extracts" further contemplates the use of a solvent known to practitioners in this art for the purpose of extraction and or formulation, including but not limited to the use of one or more solvents such as water, an alcohol, a polyhydric alcohol, an ether, an ester, a carboxylic acid, an amide, a carbonate, supercritical fluid carbon dioxide, an ionic liquid, an alkane, a petroleum-derived oil, a plant oil, or another solvent, wherein the solvent employed is optionally part of a pH-adjusted medium. The term "plant extract" or "plant extracts" as used herein further contemplates that the extract may have been obtained by the use of one or more methods such as expression, absorption by steeping or otherwise, maceration, distillation, evaporation optionally with vacuum enhancement, freeze drying, and other methods known in the arts of substance isolation from natural sources.

In some embodiments, the disclosed preparations comprise a mixture of plant extracts comprising: *Achillea millefolium* (Yarrow); *Althaea officinalis* (Althea mallow, or Marsh Mallow); *Avena sativa* (Oat); *Berberis vulgaris* (Barberry shrub); *Cochlearia officinalis* (Spoon Wort); *Conium maculatum* (Hemlock); *Ervum lens* (Lentil); *Hamamelis virginiana* (Virginium Hamamelis); *Hydrastis canadensis* (Orange Root); *Matricaria chamomilla* (Chamomil); *Nasturtium officinale* (Watercress); *Phytolacca decandra* (Poke Root); *Pimpinella saxifraga* (Pimpernal); *Populus alba* (White poplar); *Populus tremuloides* (Trembling poplar; Quaking Aspen, Trembling Aspen, Quakies); *Rhus toxicodendron* (Ivy); *Sambucus nigra* (Elderberry); *Sanguinaria canadensis* (Canadian Blood root); *Scrophularia nodosa* (Figwort); *Smilax medica* (Sarasaparrilla); *Tussilago farfara* (Coltsfoot); *Veronica officinalis* (Speedwell); and *Vincetoxicum officinale* (Swallow Wort).

In some embodiments the plant preparations disclosed herein comprise the plant extracts in the following amount (w/w):
*Achillea millefolium* from about 0.01% to about 15%;
*Althaea officinalis* from about 0.01% to about 15%;
*Avena sativa* from about 0.01% to about 25%;
*Berberis vulgaris* from about 0.01% to about 17%;
*Cochlearia officinalis* from about 0.01% to about 17%;
*Conium maculatum* from about 0.01% to about 30%;
*Ervum lens* from about 0.01% to about 17%;
*Hamamelis virginiana* from about 0.01% to about 25%;
*Hydrastis canadensis* from about 0.01% to about 17%;
*Matricaria chamomilla* from about 0.01% to about 17%;
*Nasturtium officinale* from about 0.01% to about 12%;
*Phytolacca decandra* from about 0.01% to about 17%;
*Pimpinella saxifraga* from about 0.01% to about 12%;
*Populus alba* from about 0.01% to about 17%;
*Populus tremuloides* from about 0.01% to about 17%;
*Rhus toxicodendron* from about 0.01% to about 25%;
*Sambucus nigra* from about 0.01% to about 15%;
*Sanguinaria canadensis* from about 0.01% to about 17%;
*Scrophularia nodosa* from about 0.01% to about 17%;
*Smilax medica* from about 0.01% to about 15%;
*Tussilago farfara* from about 0.01% to about 15%;
*Veronica officinalis* from about 0.01% to about 12%; and
*Vincetoxicum officinale* from about 0.01% to about 12%.

Throughout the present disclosure the term "about" a certain value means that a range of value±20%, and preferably a range of value±10%, is contemplated. Thus. for example. having about 20% w/w of an ingredient includes the ingredient being present between 16% and 24%, and preferably between 18% and 22%.

In some embodiments the plant preparations disclosed herein comprise *Achillea millefolium* from about 1% to about 15%; or from about 1% to about 10%; or from about 1% to about 8%; or from about 1.5% to about 7%; or from about 2% to about 6%. In some embodiments the plant preparations disclosed herein comprise *Achillea millefolium* in about 2.5%, while in other embodiments comprise about 5%.

In some embodiments the plant preparations disclosed herein comprise *Althaea officinalis* from about 1% to about 15%; or from about 1% to about 10%; or from about 1% to about 8%; or from about 1.5% to about 7%; or from about 1.5% to about 5%. In some embodiments the plant preparations disclosed herein comprise *Althaea officinalis* in about 2.0%, while in other embodiments comprise about 4%.

In some embodiments the plant preparations disclosed herein comprise *Avena sativa* from about 1% to about 25%; or from about 3% to about 20%; or from about 5% to about 17%; or from about 5% to about 15%. In some embodiments the plant preparations disclosed herein comprise *Avena sativa* in about 6.8%, while in other embodiments comprise about 14%.

In some embodiments the plant preparations disclosed herein comprise *Berberis vulgaris* from about 1% to about 17%; or from about 1% to about 15%; or from about 3% to about 12%; or from about 3% to about 10%. In some embodiments the plant preparations disclosed herein comprise *Berberis vulgaris* in about 4.1%, while in other embodiments comprise about 8%.

In some embodiments the plant preparations disclosed herein comprise *Cochlearia officinalis* from about 1% to about 17%; or from about 3% to about 15%; or from about 3% to about 12%; or from about 4% to about 10%. In some embodiments the plant preparations disclosed herein comprise *Cochlearia officinalis* in about 5.1%, while in other embodiments comprise about 10%.

In some embodiments the plant preparations disclosed herein comprise *Conium maculatum* from about 1% to about 30%; or from about 1% to about 25%; or or from about 3.5% to about 23%. In some embodiments the plant preparations disclosed herein comprise *Conium maculatum* in about 4%, in other embodiments comprise about 12.1%, while in yet other embodiments comprise about 21%.

In some embodiments the plant preparations disclosed herein comprise *Ervum lens* from about 1% to about 17%; or from about 1% to about 15%; or from about 3% to about 12%; or from about 3% to about 10%. In some embodiments the plant preparations disclosed herein comprise *Ervum lens* in about 3.7%, while in other embodiments comprise about 8%.

In some embodiments the plant preparations disclosed herein comprise *Hamamelis virginiana* from about 1% to about 25%; or from about 3% to about 20%; or from about 5% to about 17%; or from about 5% to about 15%. In some embodiments the plant preparations disclosed herein comprise *Hamamelis virginiana* in about 8.9%, while in other embodiments comprise about 13%.

In some embodiments the plant preparations disclosed herein comprise *Hydrastis canadensis* from about 1% to about 17%; or from about 3% to about 15%; or from about 3% to about 12%; or from about 3.5% to about 10%. In some embodiments the plant preparations disclosed herein comprise *Hydrastis canadensis* in about 5%, in other embodiments comprise about 6.0%, while in yet other embodiments comprise about 8%.

In some embodiments the plant preparations disclosed herein comprise *Matricaria chamomilla* from about 1% to about 17%; or from about 1% to about 15%; or from about 1.5% to about 12%; or from about 1.5% to about 10%. In some embodiments the plant preparations disclosed herein comprise *Matricaria chamomilla* in about 2.2%, while in other embodiments comprise about 8%.

In some embodiments the plant preparations disclosed herein comprise *Nasturtium officinale* from about 0.01% to about 12%; or from about 0.01% to about 10%; or from about 0.1% to about 8%; or from about 0.1% to about 5%. In some embodiments the plant preparations disclosed herein comprise *Nasturtium officinale* in about 0.9%, while in other embodiments comprise about 2%.

In some embodiments the plant preparations disclosed herein comprise *Phytolacca decandra* from about 1% to about 17%; or from about 3% to about 15%; or from about 3% to about 12%; or from about 3.5% to about 10%. In some embodiments the plant preparations disclosed herein comprise *Phytolacca decandra* in about 4%, in other embodiments comprise about 5.8%, while in yet other embodiments comprise about 8%.

In some embodiments the plant preparations disclosed herein comprise *Pimpinella saxifraga* from about 0.01% to about 12%; or from about 0.01% to about 10%; or from about 0.1% to about 8%; or from about 0.5% to about 5%. In some embodiments the plant preparations disclosed herein comprise *Pimpinella saxifraga* in about 1.1%, while in other embodiments comprise about 2%.

In some embodiments the plant preparations disclosed herein comprise *Populus alba* from about 1% to about 17%; or from about 3% to about 15%; or from about 3% to about 12%; or from about 4% to about 10%. In some embodiments the plant preparations disclosed herein comprise *Populus alba* in about 4.9%, while in other embodiments comprise about 10%.

In some embodiments the plant preparations disclosed herein comprise *Populus tremuloides* from about 1% to about 17%; or from about 3% to about 15%; or from about 3% to about 12%; or from about 4% to about 10%. In some embodiments the plant preparations disclosed herein comprise *Populus tremuloides* in about 4.8%, while in other embodiments comprise about 10%.

In some embodiments the plant preparations disclosed herein comprise *Rhus toxicodendron* from about 1% to about 25%; or from about 3% to about 20%; or from about 5% to about 20%; or from about 7% to about 20%. In some embodiments the plant preparations disclosed herein comprise *Rhus toxicodendron* in about 8.3%, while in other embodiments comprise about 17%.

In some embodiments the plant preparations disclosed herein comprise *Sambucus nigra* from about 1% to about 15%; or from about 1% to about 10%; or from about 1% to about 8%; or from about 1.5% to about 7%; or from about 1.5% to about 6%. In some embodiments the plant preparations disclosed herein comprise *Sambucus nigra* in about 1.9%, while in other embodiments comprise about 4%.

In some embodiments the plant preparations disclosed herein comprise *Sanguinaria canadensis* from about 1% to about 17%; or from about 3% to about 15%; or from about 3% to about 12%; or from about 4% to about 10%. In some embodiments the plant preparations disclosed herein comprise *Sanguinaria canadensis* in about 4.5%, while in other embodiments comprise about 9%.

In some embodiments the plant preparations disclosed herein comprise *Scrophularia nodosa* from about 1% to about 17%; or from about 3% to about 15%; or from about 3% to about 12%; or from about 4% to about 10%. In some embodiments the plant preparations disclosed herein comprise *Scrophularia nodosa* in about 4.0%, while in other embodiments comprise about 8%.

In some embodiments the plant preparations disclosed herein comprise *Smilax medica* from about 1% to about 15%; or from about 1% to about 10%; or from about 1% to about 8%; or from about 1.5% to about 7%; or from about 2% to about 6.5%. In some embodiments the plant preparations disclosed herein comprise *Smilax medica* in about 3.1%, while in other embodiments, comprise about 6%.

In some embodiments the plant preparations disclosed herein comprise *Tussilago farfara* from about 0.01% to about 12%; or from about 0.01% to about 10%; or from about 0.1% to about 8%; or from about 0.1% to about 5%. In some embodiments the plant preparations disclosed herein comprise *Tussilago farfara* in about 0.9%, while in other embodiments comprise about 2%.

In some embodiments the plant preparations disclosed herein comprise *Veronica officinalis* from about 0.01% to about 12%; or from about 0.01% to about 10%; or from about 0.1% to about 8%; or from about 0.1% to about 5%. In some embodiments the plant preparations disclosed herein comprise *Veronica officinalis* in about 0.8%, while in other embodiments comprise about 2%.

In some embodiments the plant preparations disclosed herein comprise *Vincetoxicum officinale* from about 0.01% to about 12%; or from about 0.01% to about 10%; or from about 0.1% to about 8%; or from about 0.1% to about 5%. In some embodiments the plant preparations disclosed herein comprise *Vincetoxicum officinale* in about 0.9%, while in other embodiments comprise about 2%.

In some embodiments the plant preparations disclosed herein comprise at least two plant extracts each of which is selected from the following list and each is present in the following amount (w/w): *Achillea millefolium* from about 0.01% to about 15%; *Althaea officinalis* from about 0.01% to about 15%; *Avena sativa* from about 0.01% to about 25%; *Berberis vulgaris* from about 0.01% to about 17%; *Cochlearia officinalis* from about 0.01% to about 17%; *Conium maculatum* from about 0.01% to about 30%; *Ervum lens* from about 0.01% to about 17%; *Hamamelis virginiana* from about 0.01% to about 25%; *Hydrastis canadensis* from about 0.01% to about 17%; *Matricaria chamomilla* from about 0.01% to about 17%; *Nasturtium officinale* from about 0.01% to about 12%; *Phytolacca decandra* from about 0.01% to about 17%; *Pimpinella saxifraga* from about 0.01% to about 12%; *Populus alba* from about 0.01% to about 17%; *Populus tremuloides* from about 0.01% to about 17%; *Rhus toxicodendron* from about 0.01% to about 25%; *Sambucus nigra* from about 0.01% to about 15%; *Sanguinaria canadensis* from about 0.01% to about 17%; *Scrophularia nodosa* from about 0.01% to about 17%; *Smilax medica* from about 0.01% to about 15%; *Tussilago farfara* from about 0.01% to about 12%; *Veronica officinalis* from about 0.01% to about 12%; and *Vincetoxicum officinale* from about 0.01% to about 12%.

In another aspect, not part of the claimed invention, disclosed herein are processes for obtaining a plant extract, the process comprising the step of a) mixing the plant with water and/or another solvent, b) distilling the mixture, and c) collecting the distillate.

In some embodiments not part of the claimed invention, the process further comprises the step of d) separating the aqueous phase from the oil phase and collecting the aqueous phase, after step c).

In some embodiments not part of the claimed invention, the process further comprises the step of e) adding more of the plant to the aqueous phase and fermenting the mixture, after step c) or after step d).

In some embodiments not part of the claimed invention, the process further comprises the step of f) distilling the fermented mixture and collecting the distillate, after step e).

In some embodiments not part of the claimed invention, the process further comprises the steps of g) heating the remaining plant material until most of the moisture has evaporated, after step f), and h) extracting the water soluble salts or hygroscopic salts from the dried plant material. In some embodiments, the remaining plant material is not completely dry. In other embodiments, the remaining plant material is completely dry. In further embodiments, the remaining plant material begins to char. In yet other embodiments, the remaining plant material begins to incinerate.

In some embodiments not part of the claimed invention, the process further comprises the step of i) adding the distillate of step e) to the extracted salts of step h).

In another aspect not part of the claimed invention, disclosed herein are plant extractions obtained by the above process.

The references to the methods of treatment by therapy or surgery or *in vivo* diagnosis methods in paragraphs [0059] to [0068] and in Examples 7 to 11 of this description are to be interpreted as references to compounds, compositions, pharmaceutical compositions and medicaments of the present invention for use in those methods.

In another aspect, disclosed herein are methods of treating inflammatory disorders in a subject, comprising identifying the subject in need thereof and administering to the subject a therapeutically effective amount of an plant preparation as disclosed herein.

In another aspect, disclosed herein are methods of preventing the onset of the symptoms of an inflammatory disorder in a subject, comprising identifying the subject in need thereof and administering to the subject a prophylactically effective amount of an plant preparation as disclosed herein.

In some embodiments of the above methods, the inflammatory disorder is one which is mediated by the innate immune system via toll-like receptors (TLRs). In other embodiments, the inflammatory disorder is mediated by one or more of pro-inflammatory cytokines selected from the group consisting of IL-6, IL-8, IL-10, IL-17, IL-12, IL-lβ, and TNF-α.

In some embodiments, the inflammatory disorder is selected from the group consisting of acne vulgaris, asthma, atopic dermatitis, autoimmune diseases, chronic prostatitis, glomerulonephritis, hypersensitivities, inflammatory bowel diseases, pelvic inflammatory disease, reperfusion injury, rheumatoid arthritis, sarcoidosis, transplant rejection, vasculitis, and interstitial cystitis.

In some embodiments, the autoimmune disorder is selected from the group consisting of alopecia areata, ankylosing spondylitis, autoimmune cardiomyopathy, autoimmune hemolytic anemia, autoimmune hepatitis, autoimmune inner ear disease, autoimmune lymphoproliferative syndrome, autoimmune peripheral neuropathy, auto immune pancreatitis, autoimmune polyendocrine syndrome, autoimmune progesterone dermatitis, autoimmune thrombocytopenic purpura, autoimmune uveitis, Coeliac Disease, cold agglutinin disease, Crohn's Disease, dermatomyositis, diabetes mellitus type 1, eosinophilic fasciitis, gastrointestinal pemphigoid, Goodpasture's syndrome, Graves' disease, Guillain-Barre syndrome (GBS), Hashimoto's encephalitis, Hashimoto's thyroiditis, idiopathic thrombocytopenic purpura, lupus erythematosus, Miller-Fisher syndrome, mixed connective tissue disease, myasthenia gravis, pemphigus vulgaris, pernicious anaemia, polymyositis, primary biliary cirrhosis, psoriasis, psoriatic arthritis, relapsing polychondritis, rheumatoid arthritis, Sjögren's syndrome, temporal , arteritis (also known as "giant cell arteritis"), ulcerative colitis, vasculitis, and Wegener's granulomatosis.

When afflicted with an epidermal condition, the human skin naturally works to alleviate the symptoms of the condition. For example, in most cases, redness, itching, or scaling due to some disorders improve over time, or until the next episode of a flare up. The plant preparations disclosed herein help the skin in its natural course to reduce the adverse symptoms. Without being bound to a particular theory, the present inventors believe that the present plant preparations provide the skin with the nutrients and building blocks necessary to efficiently and effectively work to reduce the symptoms.

Thus, in another aspect, disclosed herein are methods of reducing symptoms associated with a skin condition in a subject, comprising identifying the subject in need thereof and administering to the subject an effective amount of an plant preparation as disclosed herein.

In some embodiments, the symptoms associated with a skin condition include, but are not limited to, redness, itching, scaling, flaking, dry skin, acne, and the like associated with disorders such as acne vulgaris, psoriasis, eczema, or atopic dermatitis. In certain embodiments, the skin symptom is pruritis (itching), erythema (redness), or excoriations (flaking).

The term "subject" refers to an animal, preferably a mammal, and most preferably a human, who is the object of treatment, observation or experiment. The mammal may be selected from the group consisting of mice, rats, rabbits, guinea pigs, dogs, cats, sheep, goats, cows, primates, such as monkeys, chimpanzees, and apes, and humans.

The term "therapeutically effective amount" is used to indicate an amount of an plant preparation that elicits the biological or medicinal response indicated. This response may occur in a tissue, system, animal or human and includes alleviation of the symptoms of the disease being treated.

The term "prophylactically effective amount" is used to indicate an amount of an plant preparation that prevents the onset of the symptoms of an inflammatory disorder, or reduces the severity of the symptoms or frequency of the occurrence of the symptoms, of the inflammatory disorder. This response may occur in a tissue, system, animal or human and includes alleviation of the symptoms of the disease being treated. Therefore, while a prophylactically effective amount is administered when the subject is relatively symptom free or is in remission so as to lengthen the period of remission, whereas a therapeutically effective amount is administered either when the subject is suffering from active disease and it is desired to induce remission.

Treating," "treatment," or "therapy" of a disease or disorder means slowing, stopping, or reversing progression of the disease or disorder, as evidenced by a reduction or elimination of either clinical or diagnostic symptoms, using the compositions and methods of the present invention as described herein. These terms do not necessarily mean total cure. Any alleviation of any undesired signs or symptoms of the disease to any extent or the slowing down of the progress of the disease can be considered treatment. Furthermore, treatment may include acts that may worsen the patient's overall feeling of well being or appearance. Treatment may also include lengthening the life of the patient, even if the symptoms are not alleviated, the disease conditions are not ameliorated, or the patient's overall feeling of well being is not improved.

"Preventing," "prophylaxis," or "prevention" of a disease or disorder means prevention of the occurrence or onset of a disease or disorder or some or all of its symptoms.

In another aspect, disclosed herein are pharmaceutical compositions comprising the plant preparations disclosed herein and at least one pharmaceutically acceptable excipient, diluent, or carrier.

The term "excipient" refers to an inactive substance added to the pharmaceutical compositions disclosed herein. In some cases, the plant preparations disclosed herein may not by itself be easily administered and/or absorbed by the subject. In these cases the plant preparations may be dissolved into or mixed with an excipient. Excipients are also sometimes used to bulk up formulations that contain the plant preparations, to allow for convenient and accurate dosage. In addition to their use in the single-dosage quantity, excipients can be used in the manufacturing process to aid in the handling of the active substance concerned. Excipients also aid in stabilizing the formulations of the plant preparations, for example by keeping the various ingredients in solution or in suspension, preventing precipitation or crystallization, or adherence to container walls.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of a subject.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

Accordingly, disclosed herein are pharmaceutical compositions and methods for the treatment of inflammatory disorders. The plant preparations of the present invention can be delivered or administered to a mammal, (e.g., human subject), alone, or in the form of a pharmaceutical composition wherein the plant preparation is mixed with suitable carriers or excipient(s) in a therapeutically effective amount.

The plant preparations that are used in the methods disclosed herein can be administered as pharmaceutical compositions comprising the plant preparation together with one or more other pharmaceutically acceptable component. Pharmaceutical compositions can be in the form of solids (i.e., powders, granules, dragees, tablets, or pills), semi-solids (i.e., gels, slurries, or ointments), liquids, or gases (i.e., aerosols or inhalants).

Suitable routes of administration may, for example, inc1ude oral, topical, enteral, rectal, transmucosal, epidural, vaginal, transdermal, intravitreal, or intestinal administration; buccal administration; parenteral delivery, including intramuscular, subcutaneous, intravenous, intramedullary injections, as well as intrathecal, direct intraventricular, intraperitoneal, intranasal, or intraocular injections.

Suitable formulations for use in the present invention are found in, for example, Remington's Pharmaceutical Sciences, Mack Publishing Company, Philadelphia, PA, 17th ed. (1985) and Langer, Science, 249:1527-1533 (1990). The pharmaceutical compositions described herein can be manufactured in a conventional manner, e.g., mixing, dissolving, granulating, dragee-making, levigating, emulsifying, encapsulating, entrapping, or lyophilizing processes.

The plant preparations can be formulated with common excipients, diluents or carriers, and compressed into tablets, or formulated as elixirs or solutions for convenient oral administration. The agents can also be formulated as sustained release dosage forms and the like.

The pharmaceutical compositions disclosed herein may be manufactured in a manner that is itself known, e.g., by means of conventional mixing, dissolving, granulating, dragee-making, levigating, emulsifying, encapsulating, entrapping or tabletting processes.

Pharmaceutical compositions for use in accordance with the present disclosure thus may be formulated in a conventional manner using one or more physiologically acceptable carriers comprising excipients and auxiliaries, which facilitate processing of the active compounds into preparations, which can be used pharmaceutically. Proper formulation is dependent upon the route of administration chosen. Any of the well-known techniques, carriers, and excipients may be used as suitable and as understood in the art; e.g., in Remington's Pharmaceutical Sciences, above.

For injection, the agents disclosed herein may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants are generally known in the art.

The compositions disclosed herein can be formulated readily by combining the active compounds with pharmaceutically acceptable carriers well known in the art. Such carriers enable the compounds disclosed herein to be formulated as tablets, pills, dragees, capsules, liquids, gels, syrups, slurries, suspensions and the like, for oral ingestion by a patient to be treated. Pharmaceutical preparations for oral use can be obtained by mixing one or more solid excipient with pharmaceutical combination disclosed herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Pharmaceutical preparations, which can be used orally, include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present disclosure are conveniently delivered in the form of an aerosol spray presentation from pressurized packs or a nebulizer, with the use of a suitable propellant, e.g., dichlorodifluoromethane, trichlorofluoromethane, dichlorotetrat1uoroethane, carbon dioxide or other suitable gas. In the case of a pressurized aerosol the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

The compositions disclosed herein may also be formulated in rectal compositions such as suppositories or retention enemas, e.g., containing conventional suppository bases such as cocoa butter or other glycerides.

For topical administration, the compositions disclosed herein can be formulated in the form of solutions, suspensions, emulsions, syrups, gels, creams, lotions, or ointments. The presently disclosed compositions can also be formulated as ear or eye drops or patches.

The present compositions can be formualted in immediate release formulations or be formulated to be administered in extended release forms, including but not limited to 1 to 4 times daily, 1 to 4 times every 12 hours, 1 to 4 times every 24 hours, 1 to 4 times every 48 hours, 1 to 4 times every 60 hours, 1 to 4 times every 72 hours and 1 to 4 times every 90 hours. In some embodiments, the compositions are administered for one to 180 days.

Pharmaceutical compositions suitable for use in accordance with the present invention include compositions wherein the plant preparations are contained in a therapeutically effective amount. The therapeutically effective amounts for the methods of the present invention can depend on a variety of factors, including, e.g., age, body weight, general health, sex, diet, time and manner of administration, rate of excretion, drug combination, the judgment of the treating physician, and the severity of the particular affliction being treated.

### EXAMPLES

### Example 1: Herb Extraction

### Procedure A:

Herb extraction was achieved by steam distillation. Optionally, one or more of the steps of oil separation, fermentation, hygroscopic salt extraction, and cohobation was added to the steam distillation step. The procedures were as follows.

### Steam Distillation

Three liters of deionized water was poured into a 5 L distillation apparatus flask, which was then placed on a sand bath. Herb was added to the distillation apparatus flask until the flask was about half full. A filter paper was placed at the joint of the distillation apparatus before hooking up the reaction head adaptor. A 2 L receiving flask was used. Low heat was provided, sufficient for steam to cloud the distillation apparatus and adapter. After 1.5 L of distillate was collected, the heat was turned off and the apparatus was allowed to cool. The distillate was removed. One liter of deionized water was added to the distillation apparatus flask and the distillation was repeated. The distillation was repeated three times.

### Oil Separation

The distillate, containing an aqueous phase and an oil phase, was transferred into a foil drain tube. The stopcock at the end of the drain tube was opened and the water was drained off into a 5 L flask.

### Fermentation

Herb was placed into the 5 L flask containing the water after oil separation. The flask was sealed using a secure fermentation lock, and was incubated at 27 °C for two weeks. Subsequently, the water was distilled 5 times and the distillate was saved in a separate container.

### Hygroscopic salt extraction

The remaining plant material was boiled until the moisture was evaporated and the plant material began to incinerate. Once the incinerated plant material became grey, it was removed from heat and allowed to cool. A Soxhlet extractor was used to extract the water soluble salts from the incinerated plant material. The salts were isolated by rotary evaporation. The extracted salts were placed in a calcining kiln at 600 °C for 1 week. The process was repeated for a total of 3 times.

### Cohobation

The solution obtained after fermentation and the volatile oils were added to the isolated salts. The mixture was incubated at 30 °C for 1 week. The plant-medicinal concentrate was separated without disturbing the sediment. The concentrate can be diluted as needed.

### Procedure B:

Each individual plant raw material was subjected to steam distillation. After distillation the leftover material was in the form of wet mass. Additional water was added and the mixture was allowed to ferment in a glass container covered with a cloth lid. Fermentation was continued for 7 days at room temperature. During the fermentation the pH was monitored. Fermentation was allowed to continue until the mixtures began to putrefy. After the fermentation was over the biomass was filtered through a cloth filter and was subjected to distillation at80-90 °C to get 30-40% of distillate of filtrate.

The leftover biomass was transferred to trays made from GI/MS and covered with cloth and was kept for drying in the shade. The drying was continued until the biomass became crisp dry, i.e., for about 15-20 days. During this time the material was turned around once in a day. After drying the material was subjected to ashing by either 1) burning the dry mass and collecting the cooled ash, which was then transferred to a crucible and heated further in an oven, or 2) incinerating the biomass by heating in a crucible at 600 °C. The ash obtained is dissolved in distilled water and subjected to further distillation. All distillates were combined together and subjected to cohobation, where the combined product is heated under reflux for some time.

### Example 2: Plant Preparations

For the experiments detailed below, a unique plant preparation (HAT-01) was used. Table 2 shows the herbs and their weight percentages within this formulation.

**Table 2: Composition of HAT-01**

| Genus | Species | Common Name | Percentage Composition |
|---|---|---|---|
| *Conium* | *maculatum* | Hemlock | 12.1 |
| *Hamamelis* | *virginiana* | Witch hazel | 8.9 |
| *Rhus* | *toxicodendron* | Ivy | 8.3 |
| *Avena* | *sativa* | Oat | 6.8 |
| *Hydrastis* | *canadensis* | Orange root | 6.0 |
| *Phytolacca* | *decandra* | Poke root | 5.8 |
| *Cochlearia* | *officinalis* | Scurvy grass | 5.1 |
| *Populus* | *alba* | White Poplar | 4.9 |
| *Populus* | *tremuloides* | Quaking Aspen | 4.8 |
| *Sanguinaria* | *canadensis* | Blood root | 4.5 |
| *Berberis* | *vulgaris* | Barberry | 4.1 |
| *Scrophularia* | *nodosa* | Figwort | 4.0 |
| *Ervum* | *lens* | Lentil | 3.7 |
| *Smilax* | *medica* | Sarsaparilla | 3.1 |
| *Achillea* | *millefolium* | Yarrow | 2.5 |
| *Aesculus* | *hippocastanum* | Horsechestnut | 2.4 |
| *Capsella* | *Bursa Pastoris* | Shepherd's Purse | 2.3 |
| *Matricaria* | *chamomilla* | Chamomile | 2.2 |
| *Althaea* | *officinalis* | Mallow | 2.0 |
| *Sambucus* | *nigra* | Elderberry | 1.9 |
| *Pimpinella* | *saxifraga* | Burnet | 1.1 |
| *Vincetoxicum* | *officinale* | Swallow wort | 0.9 |
| *Nasturtium* | *officinale* | Watercress | 0.9 |
| *Tussilago* | *farfara* | Colts foot | 0.9 |
| *Veronica* | *officinalis* | Speedwell | 0.8 |
| | | Total | 100 |

Furthermore, to compare constituent efficacy and synergism, HAT01 was also partially formulated into two: HAT-01:P1 and HAT-01:P2 as described in Tables 3 and 4, respectively. These herbs have established clinical benefit, and are considered safe for clinical use in chronic inflammatory conditions.

**Table 3: Composition of HAT-01:P1**

| Genus | Species | Percentage Composition |
|---|---|---|
| *Conium* | *maculatum* | 21 |
| *Rhus* | *toxicodendron* | 17 |
| *Cochlearia* | *officinalis* | 10 |
| *Hydrastis* | *canadensis* | 8 |
| *Phytolacca* | *decandra* | 8 |
| *Berberis* | *vulgaris* | 8 |
| *Scrophularia* | *nodosa* | 8 |
| *Smilax* | *medica* | 6 |
| *Matricaria* | *chamomilla* | 4 |
| *Pimpinella* | *saxifraga* | 2 |
| *Vincetoxicum* | *officinale* | 2 |
| *Nasturtium* | *officinale* | 2 |
| *Tussilago* | *farfara* | 2 |
| *Veronica* | *officinalis* | 2 |
| | Total | 100 |

**Table 4: Composition of HAT-01:P2**

| Genus | Species | Percentage Composition |
|---|---|---|
| *Hamamelis* | *virginiana* | 13 |
| *Avena* | *sativa* | 14 |
| *Populus* | *alba* | 10 |
| *Populus* | *tremuloides* | 10 |
| *Sanguinaria* | *canadensis* | 9 |
| *Ervum* | *lens* | 8 |
| *Achillea* | *millefolium* | 5 |
| *Aesculus* | *hippocastanum* | 5 |
| *Hydrastis* | *canadensis* | 5 |
| *Capsella* | *Bursa Pastoris* | 5 |
| *Althaea* | *officinalis* | 4 |
| *Conium* | *maculatum* | 4 |
| *Phytolacca* | *decandra* | 4 |
| *Sambucus* | *nigra* | 4 |
| | Total | 100 |

Our NMR analyses of the ingredients in HAT-01 have confirmed the relative concentrations of components, and absolute concentrations of known substances. Of note, results from ¹H-NMR spectroscopic analyses of HAT-01 showed no evidence of a corticosteroid moiety, which correlates well with our findings that no changes were observed in serum cortisol levels in mice before and after treatment with oral HAT-01, and demonstrating that HAT-01 effects are nonsteroidal.

### Example 3: Methodology for Animal Studies

### Animal s

C57Bl/6 mice were purchased from Charles River laboratories (Wilmington, MA) and bred to necessity. Mice were fed mouse diet and water ad libitum. Ethanol was purchased from Fisher Scientific (Fairlane, NJ). Oxazolone (Ox) was purchased from Sigma Chemical Co. (St Louis, MO). All animal procedures were approved by the Institutional Animal Care and Use Committee of the Oklahoma Medical Research Foundation and performed in accordance with their guidelines.

### Induction: Atopic Dermatitis Model

C57Bl/6 mice were shaved on the abdomen, and were sensitized with a single topical treatment with 10 µL of 5% Ox in ethanol. After 1 week, they were treated topically with 60 µL of 0.2% Ox to the flanks once every other day for an additional 4 weeks (total of 12 challenges). Phenotype of AD-like, chronic allergic dermatitis was evidenced by the 10th challenge. This was demonstrated by persistent pruritic chronic dermatosis, increased epidermal hyperplasia, development of parakeratotic scales, and lymphocytic infiltrates. An ethanol-treated vehicle group served as the negative control.

### Treatments: AD Model

HAT-01 was topically applied twice daily on the site of induced-lesions starting from the 12th challenge. Inductions continued throughout the course of treatment (up to day 50). On the days that treatments coincided with induction, HAT-01 treatments were applied 3 hours after mice were induced with Ox. Treatments resulted in an effective and progressive reversal of the disease course, with significant clinical response in HAT-01 treated mice compared to both untreated mice and mice treated with partial formulations of HAT-01:P1 and HAT-01:P2.

### IL-10 k/o Colitis Model

Germ-free IL-10-/-mice on the same C57Bl/6 background were obtained from Jackson laboratories. Animals were observed twice daily for weight, water/food consumption, morbidity, stool consistency, piloerection, and the presence of gross blood in feces and at the anus. Disease activity index (DAI) was calculated by assigning well-established and validated scores for parameters that are somewhat analogous to the clinical presentation of human IBD, as per Cooper et al. The following parameters were used for calculation: (a) weight loss (0 point=none, 1 point=1-5% weight loss, 2 points=5-10% weight loss, 3 points=10-15% weight loss and 4 points-more than 15% weight loss), (b) stool consistency/diarrhoea (0 points =normal, 2 points= loose stools, 4 points=watery diarrhea), (c) bleeding (0 points= no bleeding, 2, slight bleeding, 4 points, gross bleeding {by ColoScreen occult blood test (Helena Laboratories, Beaumont, TX)}. The DAI was calculated as the total of these scores: the sum of weight loss, diarrhea, and bleeding, resulting in the total DAI score ranging from 0 (unaffected) to 12 (severe colitis). Unlike chemically induced mouse colitis models, which can be synchronized at the wish of investigators, the timing for the onset of colitis in IL-10 -/-mice varies greatly between individuals, most of which develop colitis around the age of 6-9 weeks in our hands. It is therefore unrealistic to predict which mouse is going to develop colitis and when. Therefore, to achieve meaningful data of therapeutic effect using IL-10 -/- mice, we monitored all IL-10 -/- mice continuously for disease activity index (DAI) and all treatments with HAT-01 were initiated when the DAI reached ~4. Once the colitis is at this stage, it develops relatively quickly, usually reaching to DAI of ~8 in about 2 weeks. Treatment of IL-10 -/- mice with HAT-01 effectively and progressively reversed the disease course to a DAI of ~2 in 16 days, while control mice (treated with saline) reached a DAI of ~8.

Following the study, animals were sacrificed by CO₂ overdose, rapidly dissected, and the entire colon was quickly removed and gently cleared of feces. Small segments of the colon taken for histopathology and immunohistochemistry (IHC) were fixed in 4% normal buffered formalin using Shandon Excelsior automated tissue processor (ThermoFisher Scientific, Waltham. MA), embedded in paraffin, sectioned at 4-um thickness with a paraffin microtome (ThermoFisher) and mounted on to microscope slides (Fisher Scientific, Pittsburgh, PA). These paraffin embedded slides were also used in immunohistochemistry (see below). Sections were stained with haematoxylin and eosin (Richard AlIen Scientific, Kalamazoo, MI), and histological scores were blindly determined as per Obermeier et al. The following parameters were used for calculation: (a) Epithelial damage (0 point=none, 1 point=minimal loss of goblet cells, 2 points=extensive loss of goblet cells, 3 points=minimal loss of crypts and extensive loss of goblet cells, and 4 points=extensive loss of crypts) (b) Infiltration (0 point=none, 1 point= infiltrate around crypt bases, 2 points= infiltrate in muscularis mucosa, 3 points=extensive infiltrate in muscularis mucosa with edema, and 4 points=infiltration of submucosa). Histological activity index (HAI) was calculated as the sum of the epithelium and infiltration score, resulting in the total HAI score ranging from 0 (unaffected) to 8 (severe colitis).

### Experimental Sepsis

Endotoxic shock was induced in C57BL/6 mice with an intraperitoneal injection of LPS (6 mg•kg⁻¹) in a final volume of 200 µL. Mice were pretreated with HAT-01 or PBS for 4 hours before receiving a bolus intraperitoneal infusion of LPS. A sham group in which normal mice received PBS (instead of LPS) was also included. Experimental and control animals were sacrificed at 48 hours after LPS injection.

### Extraction of Proteins for Western Blot Analysis

Frozen tissue samples were homogenized in homogenization buffer (50mM Tris-HC1, pH 7.2) containing Na₃VO₄ and a protease-inhibitor cocktail (Sigma-Aldrich) using an OmniTH homogenizer (OmniInternational, Marietta, GA). Following sonication, the homogenate was centrifuged at 2000xg for 5 mins. The resulting supernatants were collected as total mucosal proteins and protein concentrations were measured using the Bio-Rad Protein Assay (Bio-Rad Inc., Hercules, CA).

### SDS-PAGE and Western Blot Analysis

Protein extraction and western blots were performed as previously described with minor modifications. Briefly, proteins were separated on 12.5 % SDS polyacrylamide gels using Bio-Rad system apparatus and blotted onto nitrocellulose membranes. Nitrocellulose membranes were incubated in PBST-milk (PBS buffer containing 0.1 % Tween-20 and 5 % milk), and followed by primary antibodies (1h) for IL-6, IL-23p19, IL-17, IFNγ, IL-12p40 and Actin (diluted with PBST-milk). Blots were then washed with PBST 3 times (10 minutes each), and subsequently incubated (1h) with corresponding fluorescently labeled secondary IRDye antibodies (Rockland Immunochemicals, Inc., Gilbertsville, PA) diluted in PBST-milk. The blots were washed 3 times (10 minutes) and the fluorescence intensity of detected protein bands was quantified by the Odyssey system (LI-COR, Lincoln, NE).

### Myeloperoxidase Activity

The activity of the enzyme myeloperoxidase (MPO), a marker of polymorphonuclear neutrophil primary granules, was determined in colonic mucosa. Colonic mucosal scrapings were suspended in potassium phosphate buffer (pH 6.0) with hexadecyl trimethylammonium bromide buffer and a Sigma cocktail of protease inhibitors. Samples were then homogenized on ice with an OmniTH homogenizer, and sonicated. Following sonication, suspensions were centrifuged at 11000g for 15 minutes at 4°C, and supernatants were diluted in potassium phosphate buffer (pH 6.0) containing 0.167 mg O-dianisidine dihydrochloride (Sigma-Aldrich) and 0.0005% (vol/vol) H₂O₂. Changes in absorbance at 450 nm were recorded with a spectrophotometer every 30 seconds over 3 minutes. MPO was expressed in units per milligram of tissue, where 1 unit corresponds to the activity required to degrade 1 µmol H₂0₂/min/mL at 24°C.

### Serum Collection and Cytokine Profiling

Blood was collected by cardiac puncture in endotoxin-free silicone coated tubes without additive. The blood samples were allowed to clot at room temperature for 30 min before centrifugation (2200xg, 4°C, 10 min) and the serum was collected and stored at -80°C until analyzed. A multiplex sandwich immunoassay from Bioplex protein array system (Bio-Rad Inc, Hercules, CA) which contains fluorescence-labeled microspheres conjugated with monoclonal antibodies specific for 16 target cytokines was used. Serum samples were thawed and run in duplicates. Antibody-coupled beads were incubated with the serum sample (antigen) after which they were incubated with biotinylated secondary (detection) antibody before finally being incubated with streptavidin-phycoerythrin. A broad sensitivity range of standards (Invitrogen, Carlsbad, CA) ranging from 1.95 -32000 pg/ml were used to help enable the quantitation of a wide dynamic range of cytokine concentrations while still providing high sensitivity. Bound molecules were then read by the Bio-Plex array reader which uses Luminex fluorescent-bead-based technology (Luminex, Austin, TX) with a flow-based dual laser detector with real time digital signal processing to facilitate the analysis of up to 100 different families of color-coded polystyrene beads and allow multiple measurements of the sample ensuing in the effective quantitation of cytokines. Analytes measured include IL-lβ, IL-2, IL-4, IL-5, IL-6, IL-10, IL-l2, IL-17, IFN-γ, TNF-α, Granulocyte-macrophage colony-stimulating factor (GM-CSF), Interferon-inducible protein (IP)-10, Keratinocyte-derived chemokine (KC), Monocyte chemoattractant protein (MCP)-l, Monokine induced by IFNγ (MIG), and Macrophage inflammatory protein (MIP)-lα (Source of antibodies: Invitrogen, Carlsbad, CA).

### Example 4: In Vitro Studies

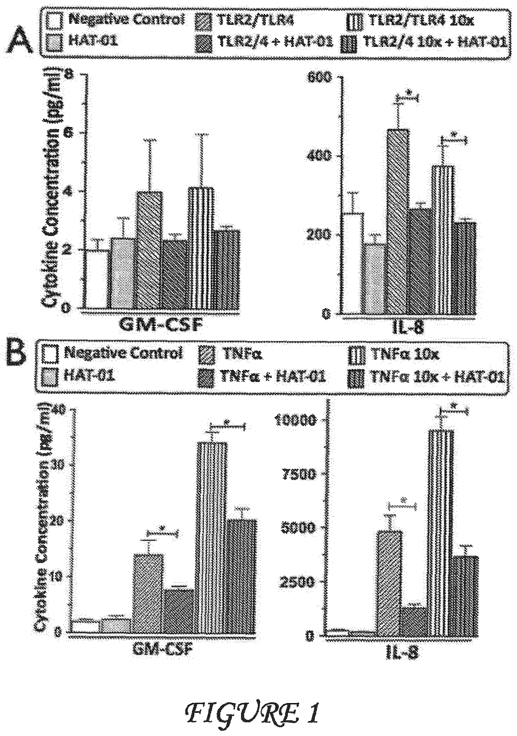

HAT-01 exerts immunomodulatory effects through its regulation of TLR functions on monocytes and keratinocytes. The chronically relapsing immunopathogenic nature of chronic inflammatory conditions has been largely attributed to the enhanced susceptibility to infections, particularly that of *S. aureus,* which has been shown to activate the innate immune system via toll-like receptors (TLRs), resulting in pro-inflammatory gene expression and cytokine secretion. Recent studies have ascribed the enhanced susceptibility to *S. aureus* in AD to impaired TLR-2 expression, dysfunctional TLR-2-mediated effects, and dysregulated IL-8 and IL-6 secretion in AD patients. To investigate the role of HAT-01 in TLR regulation. we assessed the effect of HAT-01 on the monocytic cell line Mono Mac 6 (MM6) and the human keratinocyte cell line (HaCaT), either of which did not have cytotoxic effects at the concentration of HAT-01 tested. We performed induction studies with several TLR ligands [Pam3Cys (TLR2), LPS (TLR2/TLR4), Flagellin (TLR5). FSL (TLR6), and lmiquimod (TLR7) and TNF-α]. As shown in Fig. 1, our studies demonstrated that HAT-01 modulates TLR2, TLR4, TLR5 and TNFα, which was exhibited by a predominant decrease in proinflammatory cytokines. Treatment with HAT-01 alone (no induction) did not significantly alter GM-CSF or IL-8 production in these cells. This indicated the novel finding that HAT-01 may exert its immunomodulatory effects, in part by modulating TLR2, TLR4 functions, and reducing proinflammatory cytokine secretion.

### Example 5: Mouse Studies for Inflammatory Bowel Disease and Sepsis

### HAT-01 significantly suppresses mice from developing systemic inflammation in murine models of colitis.

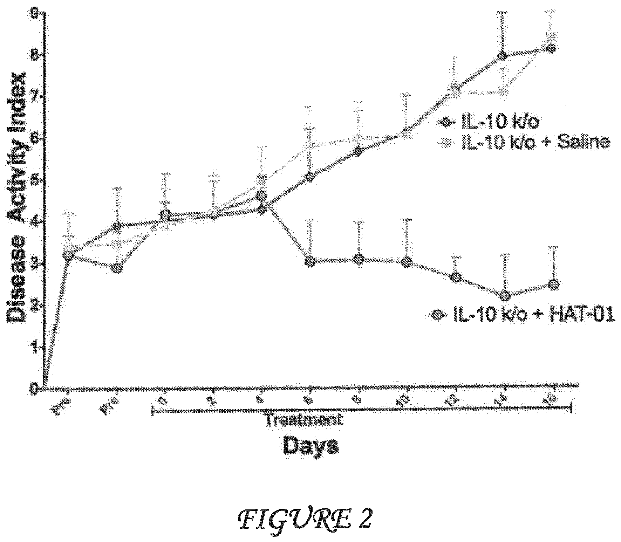

To further evaluate the immunomodulatory potential of HAT-01, we chose to evaluate the anti-inflammatory and immunomodulatory role of HAT-01 in two well-established models of inflammation: a spontaneously developing IL-10 -/- model of colitis, and a model of experimental sepsis. IL-10 deficient mice model [IL-10-/-], purchased from Jackson Laboratories, which spontaneously develop colitis is one of the most commonly used genetic model of colitis. Unlike chemically induced mouse colitis models, which can be synchronized at the wish of investigators, the timing for the onset of colitis in IL-10 -/-mice varies greatly between individuals, most of which develop colitis around the age of 6-9 weeks in our hands. It is therefore unrealistic to predict which mouse is going to develop colitis and when. Therefore, to achieve meaningful data of therapeutic effect using IL-10 -/-mice, we monitored all IL-10 -/-mice continuously for disease activity index (DAI) and all treatments with HAT-01 were initiated when the DAI reached ~4. Once the colitis is at this stage, it develops relatively quickly, usually reaching to DAI of ~8 in about 2 weeks (Fig. 2). As shown in Fig. 2, treatment of IL-10 -/-mice with HAT-01 effectively and progressively reversed the disease course to a DAI of ~2 in 16 days, while control mice (treated with saline) reached a DAI of ~8. This was also evidenced both morphologically and histologically, where IL-10 -/-mice treated with HAT-01 demonstrated significant protection from the effects of colitis and have normal colonic mucosa with little signs of inflammation (data not shown).

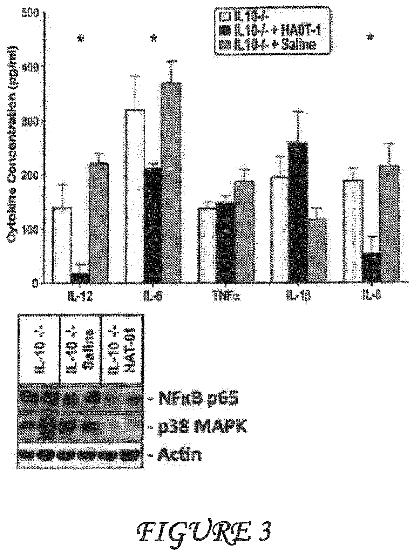

Profiling reveals a HAT-01-distinct immunosuppressive cytokine pattern, mediated by NFκB & MAPK. To address the molecular mechanisms of HAT-01 therapeutic effects in systemic inflammation, we analyzed systemic cytokine profiles and tissue-specific signaling mediators of the adaptive immune response in IL-10 -/- mice. Our studies demonstrated that treatment with HAT-01 greatly increases the production of systemic anti-inflammatory mediators, while suppressing pro-inflammatory mediators (Fig. 3 Graph). Furthermore, HAT-01 treatment decreased the activation (phosphorylation) of NFκBp65 and p-MAPK in colonic mucosa when compared to untreated mice, suggesting the serum cytokine levels are closely correlated with those in the colonic mucosa, and further demonstrating the immunosuppressive profile of HAT-01 in its therapeutic effects on systemic inflammation (Fig. 3 Gel).

### HAT-01 significantly suppresses mice from developing systemic inflammation in murine models of experimental sepsis.

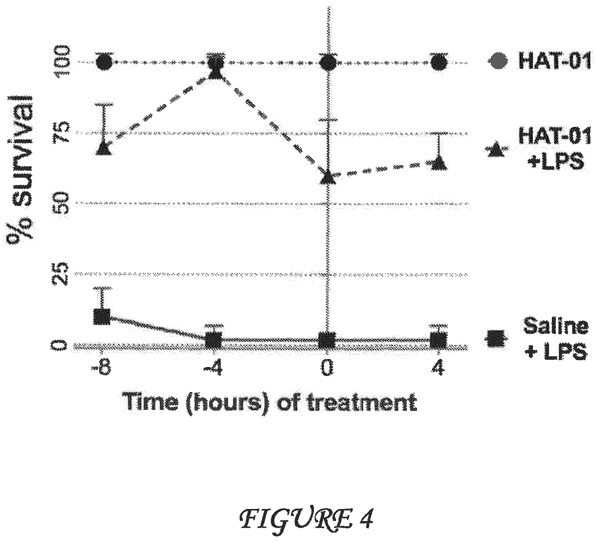

Additionally, to establish a model of systemic inflammation, a model of endotoxemia was induced in C57Bl/6 mice following LPS administration. Mice were pretreated with HAT-01 for 4 hours before receiving a bolus intraperitoneal infusion of LPS (6 mg/kg). A sham group in which normal mice received saline in place of LPS was also included. Mice were followed up to 48 hours post-treatment, at which time mice from the sham group and the LPS groups were sacrificed and examined. As shown in Fig. 4, pretreatment with HAT-01 afforded substantial protection (71%) at the earliest time-point tested, and demonstrated ~100% protection when pretreated at 4 hours before LPS administration. Treatment of endotoxemia was also demonstrated when mice were treated at 4 hours after LPS administration, suggesting that HAT-01 was able to significantly suppress mice from developing systemic inflammation.

### Example 6: Atopic Dermatitis

Atopic Dermatitis (AD), the most common cause of chronic inflammatory skin diseases, is characterized by a disturbance of epidermal-barrier function resulting in intensely pruritic and chronic eczematous plaques. While the formulations disclosed herein can be broadly applied to all chronic inflammatory conditions, AD became a focus of our studies due to: (a) the significant unmet medical need where the flares are treated through symptomatic management with topical corticosteroids, which come with long-term undesirable and potentially serious side-effects, and response attenuation in some patients, (b) the potential of treating AD with a topical plant formulation that could positively impact safety and efficacy, and (c) the observed suppression of TLR activation by this plant formulation could provide an adjunct to conventional AD therapies, which do not directly target this pathway.

We evaluated the immunomodulatory and therapeutic potential of this plant preparation in several *in vitro* cell-lines and found it to demonstrate no cytotoxic activity on non-immune cells, but exhibit profound immunosuppressive effects on immune-cells. We also characterized the immunosuppressive profiles of this plant formulation, and have identified this to be mediated through specific toll-like receptor (TLR)-modulated mechanisms. Our *in vitro* studies using this plant formulation were followed with an assessment of efficacy where we found that it significantly suppresses mice from developing systemic inflammation in murine models of colitis, experimentally-induced sepsis, and mouse models of AD, where we observed significant therapeutic benefit. Based on these promising findings, we undertook two independent clinical trials: an open-label pilot trial and a randomized, single (patient) blind, placebo-controlled trial to assess efficacy and tolerability of a topical formulation of this plant formulation in patients with AD. The clinical response was striking with significant improvement in clinical signs and symptoms in the majority of patients treated with this plant formulation. Response was seen within 4 weeks of treatment with this plant formulation, with sustained effects and no relapses at 8-week or 12-weeks of follow up, when compared to both placebo and treatment with partial formulations of this plant formulation. Our studies have exploited sophisticated technologies into component-based and mechanistic studies of plant medicine, thereby contributing to the advancement and modernization of plant medicine.

### Novel therapeutic effects of HAT-Cl in murine chronic atopic dermatitis.

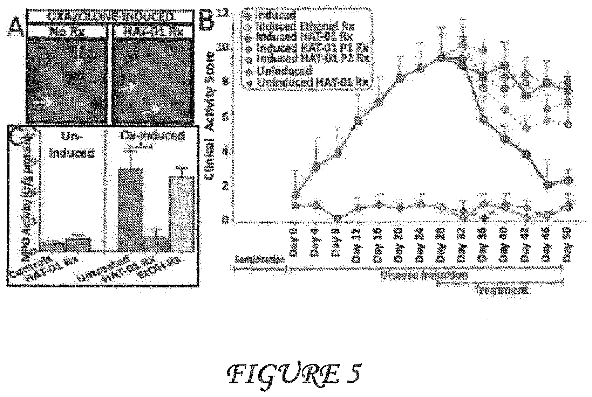

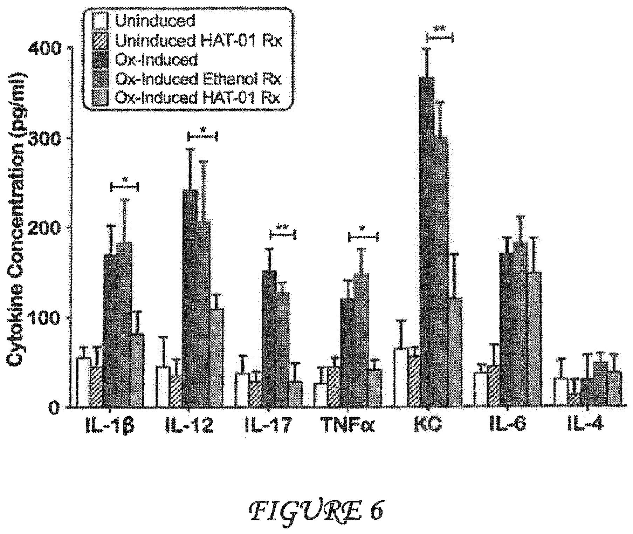

Given the above results, we proceeded to use an *in vivo* model where we tested the therapeutic effects of HAT-01 on the well-established oxazolone-induced murine model of chronic AD. Mice were sensitized with a single topical application of 5% oxazolone (Ox) in ethanol to the flank, followed by challenges with topical 0.2% Ox every other day for 4 weeks (12 challenges). An ethanol-treated vehicle group served as the negative control. Ox treated mice developed chronic atopic dermatitis as evidenced by persistent pruritic chronic dermatosis (Fig. 5), increased epidermal hyperplasia, development of parakeratotic scales, and lymphocytic infiltrates. Treatment with twice-daily topical applications of HAT-01 on the site of induced-lesions effectively and progressively reversed the disease course, with significant clinical response in HAT-01 treated mice compared to both untreated mice and mice treated with partial formulations of HAT-01:P1 and HAT-01:P2 (indicated in Fig. 5B). Response was evidenced by a mean 73.4% decrease in signs of pruritis, edema, erythema, and excoriations when compared to untreated mice (Fig. 5). Effects were also observed histologically and enzymatically. HAT-01 treated mice skin biopsies had significantly decreased dermal inflammatory infiltrates and hyperplasia, and a mean 82.2% decrease in dermal myeloperoxidase activity, a marker of innate neutrophil infiltration, than vehicle treated mice (Fig 5C). At the systemic level, when compared to induced-mice, treatment with HAT-01 significantly decreased pro-inflammatory cytokines IL-17, IL-12, IL-lβ, TNF-α, and the murine IL-8 homologue KC only in the Ox-induced animals, further indicative of the immunosuppressive potential of HAT-01 in AD (Fig 6). No significant changes in levels of these cytokines were observed in Ox-induced mice treated with the vehicle alone, or in HAT-01 treated uninduced mice.

### Example 7: Clinical Studies

### Methodology

The clinical trial was conducted in a contract research organization setting with three investigators (dermatologists) in a multi-center site. Patients were block randomized from a central location into each study arm. Patients were assessed both before and throughout the course of treatment at each visit using by SCORAD and a Patient Benefit Index (which assesses AD patient-relevant treatment benefit). SCORAD values, ranging from 0 to 103, are classified as mild <15), moderate (16-40) and severe (>41), and are calculated as (0.2 x Area) + (3.5 x Erythema + Edema + Crust + Excoriation + Lichenification + Dryness) + (pruritis + sleep loss). All three investigators were given prior training and testing for standardized scoring of SCORAD in AD, an approach that has been found to be significantly beneficial for minimizing observer variability.

Our primary objective was the efficacy of HAT-01, HAT-01P1, and/or HAT-01P2 in the reduction of pruritus in atopic dermatitis patients.

Our secondary objective(s) were to
(a) evaluate the therapeutic benefit of HAT-01, HAT-01P1, and/or HAT-01P2 in atopic dermatitis as measured by the SCORAD (SCORing Atopic Dermatitis),
(b) evaluate the therapeutic benefit of HAT-01, HAT-01P1, and/or HAT-01P2 to reduce pruritus from patient's perspective as assessed by the Patient Benefit Index, and
(c) safety and tolerability of HAT-01, HAT-01P1, and/or HAT-01P2 in atopic dermatitis patients.

Double Blinded Randomization: A double blind approach where both investigators and patients are blinded was used. Investigators performed enrollment and blinded assignment using blinded labels. Assignments were rotated through enrollment by investigators to enable random continuous enrollment and avoid selection bias.

Treatment: Atopic dermatitis patients fulfilling eligibility (inclusion and exclusion criteria) were provided with an informed consent. A topical formulation of HAT-01, HAT-01P1, and/or HAT-01P2 was randomly and blindly provided (without patient knowledge) and followed through 2, 4, 8, and 12 weeks after treatment. Patients were assessed both before and throughout the course of treatment (each visit) for the following indices: SCORAD (SCORing Atopic Dermatitis), Patient Benefit Index. Each patient also had up to 10 mls of blood drawn prior to (0) and at 4, and 12, weeks post-therapy to distinguish laboratorial and cytokine changes over time. Serum from 64 patients with AD on an approved randomized single blind placebo-controlled clinical trial of HAT-01, placebo and partial formulations of HAT-01 were obtained for all groups at 3 time points (0, 4, 12 weeks).

Inclusion criteria included:
(a) clinical diagnosis of atopic dermatitis according to Hanifin and Rajka,
(b) treatment area amenable to topical treatment,
(c) attending a hospital outpatient clinic or the private practice of a dermatologist,
(d) following verbal and written information about the trial, the patient must provide signed and dated informed consent before any study related activity is carried out, including activities relating to washout period,
(e) males or females between 14-60 years, with adequate consideration to minorities and children (guardian permission required).

Exclusion criteria included:
(a) systemic treatment with immunosuppressive drugs or corticosteroids within 4 weeks prior to enrollment, (Inhaled or intranasal steroids for asthma or rhinitis may be used),
(b) topical treatment with immunomodulators or corticosteroids within 4 weeks prior to enrollment,
(c) phototherapy therapy within 4 weeks prior to enrollment,
(d) other topical therapy on the treatment area within 1 week prior to enrollment,
(e) use of anti-histamine treatment during the study,
(f) clinical infection, (impetiginised atopic dermatitis) on the treatment area,
(g) patients with history of cancer including skin cancer,
(h) patients with history of an immunocompromised disease,
(i) current participation in any other interventional clinical trial,
(j) pregnancy or risk of pregnancy, and/or lactation,
(k) history of allergy of any plant components,
(l) Subjects with intense sun exposure during the study,previously treated with HAT-01, HAT-01P1, and/or HAT-01P2,
(n) patients known or suspected of not being able to comply with a trial protocol, (e.g. alcoholism, drug dependency, or psychotic state).

### Therapeutic effects of HAT-01 in patients with atopic dermatitis: Pilot Trial

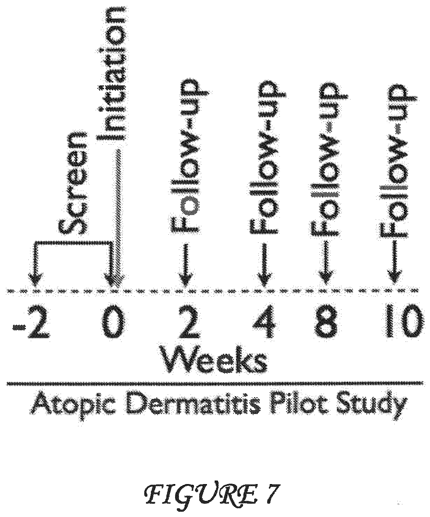

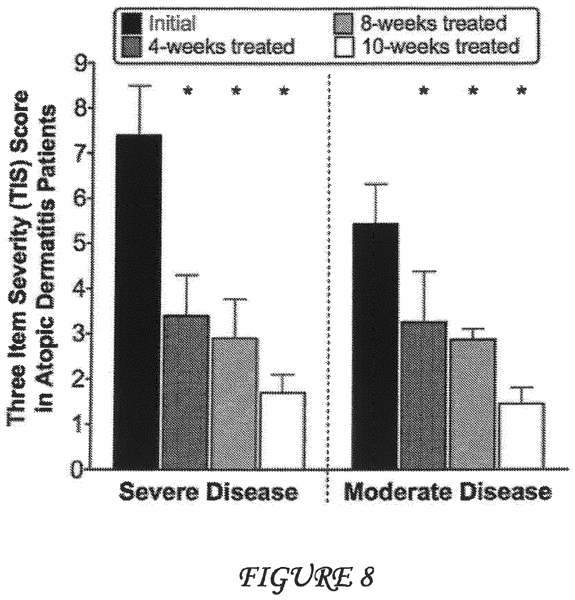

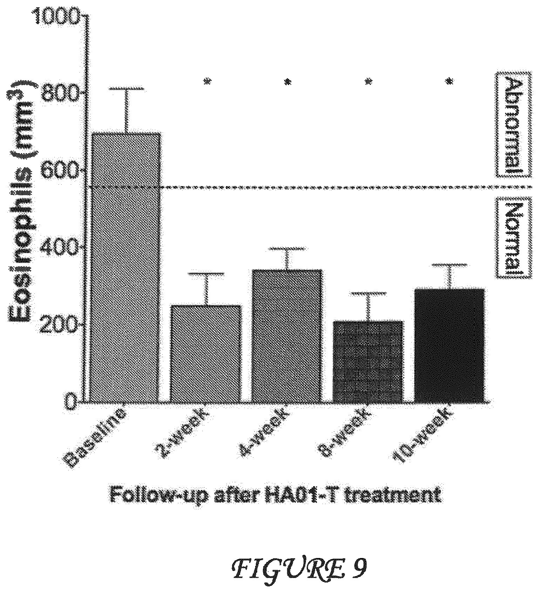

To correlate our findings in human patients with AD, we performed an exploratory 10-week, open-label study to evaluate the utility of HAT-01 in atopic dermatitis. Significant resolution of symptoms and signs of AD was observed through a Three Item Severity score in 15 of 18 patients after 4 weeks of topical HAT-01 treatment, with no relapses at 10-weeks of follow up. We then performed pilot clinical studies to evaluate the utility of HAT-01 in atopic dermatitis. Patients with severe asthma and/or those treated with systemic or topical (greater than medium strength) corticosteroids or immunosuppressant agents were excluded from the study. An exploratory 10-week, open-label study was designed to investigate the safety, efficacy and tolerability of a twice-daily application of a topical formulation of HAT-01 in AD. A total of 18 patients fulfilling diagnostic criteria for AD were enrolled and placed on a regimen of twice daily application of a topical formulation of HAT-01 (300 µl of 1:10). Fig. 7 portrays the design outline of this pilot trial. The primary efficacy outcome was defined as the change in the Three Item Severity score (TIS score) of AD based on the evaluation of erythema, oedema/papulation and excoriation in each representative lesion. Significant resolution of symptoms and signs of AD was observed in 83% of patients (15 of 18) after 4 weeks of HAT-01 treatment, with no relapses at 8-week or 10-week follow up (Fig 8). Patients with severe disease activity pretreatment (TIS score > 6) demonstrated an average 54.6% reduction in disease activity at 4-weeks, and an average of 77.1% reduction in disease activity by 10-weeks (Fig 8). Of note, a significant reduction in edema and erythema was observed within 2-weeks, which was followed by improvements in oozing and excoriations within 4 weeks following treatment with HAT-01. Significant improvement in xerosis (dryness) was also immediately observed, while improvement in lichenification (thickening) took up to 10 weeks for effective resolution. Of the 11 patients that also presented with eosinophilia (>500 eosinophils/mm3), significant reduction to within normal levels was observed in all 11 patients (100%) within 2-weeks following HAT-01 treatment. Significant decreases in peripheral blood eosinophils at all post-baseline visits were also observed in these 11 patents (Fig 9).

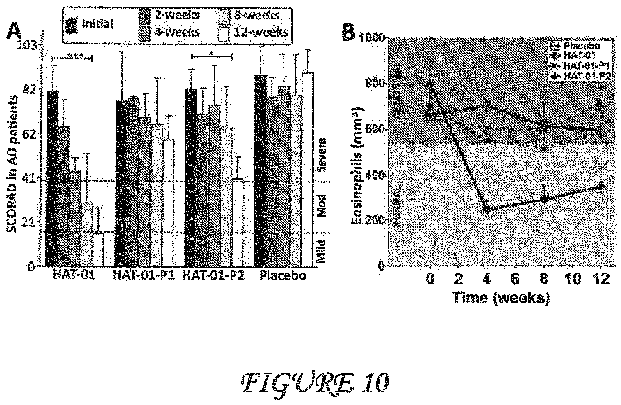

AD: Therapeutic effects of HAT-01 in patients with atopic dermatitis: Randomized placebo-controlled trial To further investigate the safety, efficacy and tolerability of a twice-daily application of a topical formulation of HAT-01 in atopic dermatitis (AD), a total of 64 patients fulfilling inclusion criteria for AD were enrolled into a randomized placebo-controlled single (patient) blind clinical trial with a regimen of twice daily application of a topical formulation of HAT-01 (300µl of 1:10). The primary efficacy outcome was defined as the change in the Severity Scoring of Atopic Dermatitis (SCORAD) values. SCORAD values, ranging from 0 to 103, are classified as mild (<15), moderate (16-40) and severe (>41), and are calculated as (0.2 x Area) + (3.5 x Erythema + Edema + Crust + Excoriation + Lichenification + Dryness) + (pruritis + sleep loss). As shown in Fig 10A, significant resolution of symptoms and signs of AD was observed in 84% of patients after 4 weeks of HAT-01 treatment, with sustained effects and no relapses at 8-week or 12-weeks of follow up (Fig 10A). Placebo (vehicle) treatment had no significant effect on SCORAD values, and treatment with partial formulations HAT-01:P1 and HAT-01:P2 had only minimal effects relative to treatment with whole HAT-01 (Fig 10A). Significant reduction in edema and erythema was observed within 4-weeks, which was followed by improvements in oozing and excoriations within 8 weeks following treatment with HAT-01. Significant improvement in xerosis was also observed at 4 weeks, while improvement in lichenification took up to 12 weeks for effective resolution. Significant decreases in peripheral blood eosinophilia at all post-baseline visits to normal levels were also observed in patients treated with HAT-01 when compared to those treated with partial formulations and/or placebo (Fig 10B). Since eosinophils play an important role as a lymphocyte chemoattractant in AD, and given that eosinophilia is an important diagnostic criterion in AD, these results further support the hypothesis that HAT-01 significantly suppresses skin inflammation in AD in a clinically relevant manner. Importantly, HAT-01 was well tolerated and no treatment-related adverse events were observed throughout the 12-week pilot trial, indicating the safe and potent therapeutic benefit of HAT-01 in a majority of the patients studied with AD.

### Comparison of therapeutic effects of HAT-01 between human and mouse AD studies.

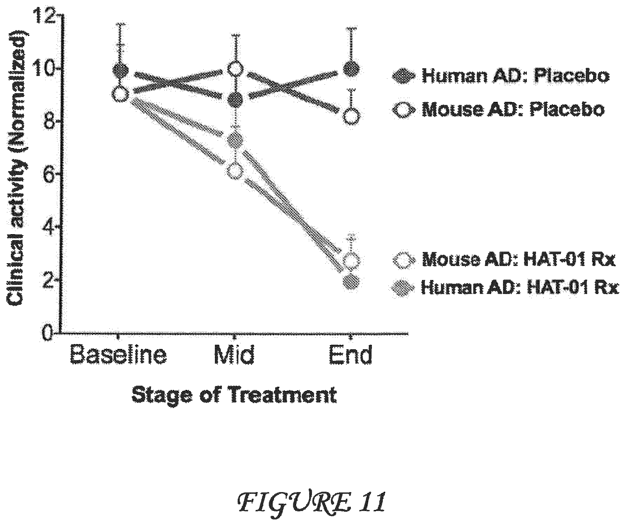

To assess the similarities of HAT-01 response between our human clinical trials and our murine models of AD, we compared the clinical SCORAD (human) and clinical activity (mouse) scores for three time points (baseline, mid and end) from our studies normalized to the range in scores observed (Fig 11). When compared to placebo, the clinical response to HAT-01 in the murine model of eczema/AD is strikingly similar to response in human AD, indicating that the murine model can serve as effective correlate for the development of human biological assays for product stability and potency.

### Example 8: Case Reports: HAT-01 Experience

### Case 1

A 47 year old previously healthy male presented to the clinic with chief complaint of fever, myalgia, sore throat, mildly productive cough with yellow sputum, pleuritic chest pain, and increasing shortness of breath for ten days. He is an active smoker with a 30-year smoking history. He had no significant travel, occupational, or exposure history. On physical examination, the patient was found to be febrile and had bilaterally diminished breath sounds and bilateral coarse crackles in the lower chest area. The sputum smear was positive for acid fast bacilli. With the exception of total leukocyte count which were elevated at 18000 cells per cubic millimeter, other laboratory study findings including blood chemistry measurements were normal. Chest x-ray confirmed pneumonic infiltrate in the lower one-third area of the right lung and a diagnosis of community-acquired pneumonia was made. The patient was treated with a half-teaspoon three times daily oral dose of HAT-01 for 1 week. At the 2-week follow-up, the patient reported complete resolution of symptoms. Physical examination demonstrated no evidence of residual signs and a repeat laboratory total leukocyte count depicted normal levels at 10000 cells per cubic millimeter. Given these findings, our studies indicate that HAT-01 has potent therapeutic effects in acquired pneumonia.

### Case 2

A 51 year old male with a history of type 2 diabetes mellitus for 9 years was referred by his general practitioner. The patient presented with the chief complaint of two ulcers: a persistent right plantar ulcer with foul smelling discharge and one superficial ulcer over the dorsum of the right foot. Past history of the patient was significant for peripheral neuropathy, and recurrent plantar ulcers. On physical examination, the patient had mild edema in the distal lower extremities. Both dorsalis pedis and posterior tibialis pulses were palpable with feeble pulses. The plantar ulcer was a Grade 3 ulcer and measured 8 x 4 x 2 cms. The superficial ulcer on the lateral malleolus was a Grade 1 ulcer and measured 4 x 3.5 x 0.5 cms. (The Wagner Grading System was used). No evidence of osteomyelitis was evident. Abnormalities in the complete blood work from the laboratory tests revealed elevated total leukocyte cell count (15700 cells per cubic millimeter), and fasting blood glucose at 142 mg/dL. Radiographs did not indicate osseous impairment. The patient was on 30 units of Humulin-R insulin prior to presentation. A diagnosis of type 2 diabetes mellitus with a Grade 1 to 3 diabetic foot ulcer was made. After sharp debridement of necrotic tissue, the patient was treated with four times daily topical application of HAT-01 in 1: 10 dilution, and dressings were applied. The patient was asked to follow-up with biweekly visits to the clinic. Granulation tissue and healing was visibly evident from the first follow-up visit at 2 weeks, and the ulcer significantly decreased in size over the course of the next few weeks. Six weeks after initial treatment, both wounds had completely healed. The patient has continued to remain free of ulceration at the time of this writing (10 months later), suggesting that HAT-01 has potent therapeutic effects in diabetic foot.

### Example 9: Therapeutic effects of HAT-01 in patient with psoriasis: Pilot Trial

To assess the effects of HAT-01 in psoriasis, we performed an 8-week, open-label, two armed study (HAT-01 and OTC medication arm) in patients with mild to moderate chronic plaque psoriasis. Patients (aged 18 to 70 years) with stable chronic plaque psoriasis of at least 6 months duration with a psoriasis body surface area (BSA) ≤ 10% were included. All areas with the exception of the face, groin and axillae were treated. Patients with current or recent malignancies, severe asthma, infections, uncontrolled hypertension, and/or those treated with systemic or topical corticosteroid or immunosuppressant agents were excluded from the study. Patients with abnormal screening laboratory values, as well as pregnant women, were also excluded from the study. Patients were not allowed to use any medicated cosmetics that might influence the outcome of the study. A total of 16 patients fulfilling psoriasis inclusion criteria were enrolled into an exploratory 8 week, open-label study designed to investigate the safety, efficacy and tolerability of HAT-01. Patients were enrolled into one of two arms: (a) twice-daily application of HAT-01 (n=9 patients) or (b) twice-daily application of over-the-counter topical medications which are first-line therapy for patients with psoriasis and included topical vitamin D₃ analog calcipotriene or topical retinoids (n=7 patients). Approximately 300µl of HAT-01 was applied per lesion, prepared as a 10% dilution of the concentrated plant extracts in 4.9% EtOH. The primary efficacy outcome was defined as the percentage change from baseline in the body surface area affected by psoriasis at weeks 2, 4, and 8 following treatment. Safety evaluations included tolerability assessments and incidence of adverse events.

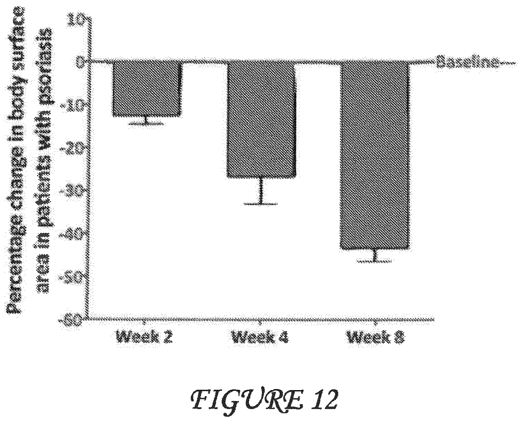

Patients were evaluated for changes in total body surface area, erythema, scaling, and plaque resolution. Patients treated with HAT-01 with moderate disease activity pretreatment demonstrated an average 42% reduction in the percentage in body surface area affected by psoriasis at 8 weeks, whereas those treated with a control OTC topical vitamin D₃ analog calcipotriene and topical retinoids exhibited a reduction in the percentage in body surface area affected by psoriasis by only 12% (Fig 12). Significant resolution in erythema and scaling with changes in plaque elevation (from that of marked to slight) was observed in 8 of 9 patients after 8 weeks of HAT-01 treatment.

HAT-01 was well tolerated, with no treatment-related adverse events observed throughout the 8-week trial. At week 8, all patients treated with HAT-01 had no reports of skin atrophy, pruritus, or burning/stinging. In contrast at 8 weeks of treatment, 4 of 7 patients that were treated with control OTC topical vitamin D₃ analog calcipotriene exhibited local treatment-site irritation, burning sensation, and pain.

### Example 10: Therapeutic effects of HAT-01 in patients with acne vulgaris: Clinical vignettes

To assess the effects of HAT-01 in acne vulgaris, an open-label study of HAT-01 in subjects with mild to moderate (grade I and II) acne vulgaris was undertaken at an outpatient setting of a private practice clinic. Subjects aged 18 to 50 years of age with ≥ three inflammatory or non-inflammatory lesions of papules or pustules in the face were included. Exclusion criteria included: severe acne, the use of any anti-acne medications within the preceding four weeks, or subjects with cystic lesions or any acne requiring systemic treatment. Subjects were not allowed to use any medicated cosmetics that might influence the outcome of the study. A total of 4 patients fulfilling criteria were enrolled. The primary efficacy outcome was defined as the change from baseline of the total lesion count. Subjects were asked to follow a 4 week regimen of twice daily applications of HAT-01 (300µl applied per lesion, prepared as a 10% dilution of the concentrated plant extracts in 4.9% EtOH). An analysis of the post-treatment inflammatory and non-inflammatory lesion counts demonstrated a highly significant reduction of scores from baseline to 4 week time points, indicative of the therapeutic potential of HAT-01 in acne vulgaris. HAT-01 was well tolerated, with no treatment-related adverse events observed throughout the 4-week trial including no reports of skin atrophy, pruritus, or burning/stinging at the local treatment-site.

### Example 11: Therapeutic effects of HAT-01 when compared to commonly used AD therapies in a murine AD model.

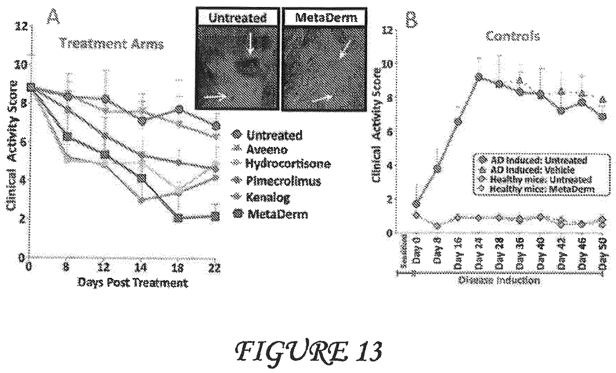

We utilized a well-established AD animal model (the oxazolone-induced murine model of chronic AD) to test the therapeutic effects of HAT-01. Mice (n=10) were sensitized with a single topical application of 5% oxazolone (Ox) to the flank, followed by challenges with 0.2% topical Ox every other day for total of 4 weeks. An ethanol-treated vehicle group served as the unaffectected control. Induced mice developed chronic AD as evidenced by persistent pruritic chronic dermatosis and increased epidermal hyperplasia, development of parakeratotic scales, and lymphocytic infiltrates (Fig. l3). A comparator-based efficacy study was performed in this model to test the therapeutic effects of HAT-01 relative to that of commonly available AD therapies. Comparators used included twice-daily applications of 0.5% triamcinolone acetonide cream (Kenalog), 1% pimecrolimus cream, 1% hydrocortisone OTC cream, and 1% colloidal oatmeal OTC cream (Aveeno Eczema Therapy). A Clinical Activity Score, equal to the sum of the severity of 5 signs and symptoms (itch, erythema/hemorrhage, edema, excoriation/erosion and scaling/dryness), each of which are graded on a 3-point scale (0 - none, 1 - mild, 2 - moderate, 3 - severe) was used to evaluate therapeutic efficacy. At day 22 post-treatment, mean Clinical Activity Scores were decreased by 75.93% for HAT-01 (Fig. 13A). This level of efficacy was significantly higher than the other AD therapies tested including: Aveeno (p < 0.0001), Hydocortisone (p =0.0007), Pimecrolimus (p =0.002), and Kenalog (p =0.019) (Fig 13A). HAT-01 also decreased histological and biochemical aspects of AD in this model. Skin biopsies in HAT-01-treated mice had significant decreases in hyperplasia and dermal inflammatory infiltrates. No abnormal findings were observed in the morphological and histological investigations of all tissues from AD mice that were treated with HAT-01.

### List of References:

1. Matthews, HB, Lucier, GW, Fisher, KD, Medicinal herbs in the United States: research needs. Environ Health Perspect 1999;107(10):773-8.
2. Tindle, HA, Davis, RB, Phillips, RS et al., Trends in use of complementary and alternative medicine by US adults: 1997-2002. Altern Ther Health Med 2005; 11(1):42-9.
3. Normile, D, Asian medicine. The new face of traditional Chinese medicine. Science 2003;299(5604): 188-90.
4. Iauk, L, Lo Bue, AM, Milazzo, I et aI., Antibacterial activity of medicinal plant extracts against periodontopathic bacteria. Phytother Res 2003; 17(6):599-604.
5. Guo, R, Pittler, MH, Ernst, E, Complementary medicine for treating or preventing influenza or influenza-like illness. Am J Med 2007; l20(11):923-9.e3.
6. Cwikla, C, Schmidt, K, Matthias, A et al., Investigations into the antibacterial activities of phytotherapeutics against Helicobacter pylori and Campylobacter jejuni. Phytother Res 2009
7. Attardo, C, Sartori, F, Pharmacologically active plant metabolites as survival strategy products. BoIl Chim Farm 2003;142(2):54-65.
8. Wolff, HR, Kieser, M, Hamamelis in children with skin disorders and skin injuries: results of an observational study. Eur J Pediatr 2007;166(9):943-8.
9. Horii, KA, Simon, SD, Liu, DY et al., Atopic dermatitis in children in the United States, 1997-2004: visit trends, patient and provider characteristics, and prescribing patterns. Pediatrics 2007;120(3):e527-34.
10. Leung, DY, Bieber, T, Atopic dermatitis. Lancet 2003;361(9352):151-60.
11. Abramovits, W, Perlmutter, A, Steroids versus other immune modulators in the management of allergic dermatoses. Curr Opin Allergy Clin Immunol 2006;6(5):345-54.
12. McClain, RW, Yentzer, BA, Feldman, SR, Comparison of skin concentrations following topical versus oral corticosteroid treatment: reconsidering the treatment of common inflammatory dermatoses. J Drugs DermatoI2009;8(12):1076--9.
13. http://www.naturalstandard.com/
14. Takeuchi, 0, Hoshino, K, Kawai, T et al., Differential roles of TLR2 and TLR4 in recognition of gram-negative and gram-positive bacterial cell wall components. Immunity 1999;11(4):443-51.
15. Niebuhr, M, Lutat, C, Sigel, S et al., Impaired TLR-2 expression and TLR-2-mediated cytokine secretion in macrophages from patients with atopic dermatitis. Allergy 2009
16. S. Wirtz, M.P. Neurath, Mouse models of inflammatory bowel disease, Adv Drug Deliv Rev 59 (2007) 1073-1083.
17. L.F. Poli-de-Figueiredo, A.G. Garrido, N. Nakagawa, P. Sannomiya, Experimental models of sepsis and their clinical relevance, Shock 30 Suppl 1 (2008) 53-59.
18. Hatano, Y, Man, MQ, Uchida, Y et al., Murine atopic dermatitis responds to peroxisome proliferator-activated receptors alpha and beta/delta (but not gamma) and liver X receptor activators. J Allergy Clin ImmunoI201O;125(l):160-9.e1-5.
19. Man, MQ, Hatano, Y, Lee, SH et al., Characterization of a hapten-induced, murine model with multiple features of atopic dermatitis: structural, immunologic, and biochemical changes following single versus multiple oxazolone challenges. J Invest DermatoI2008;128(1):79-86.
20. Oranje, AP, Glazenburg, EJ, Wolkerstorfer, A et al., Practical issues on interpretation of scoring atopic dermatitis: the SCORAD index, objective SCORAD and the three-item severity score. Br J Dermatol 2007; 157(4):645-8.
21. Wolkerstorfer, A, de Waard van der Spek, FB, Glazenburg, EJ et aI., Scoring the severity of atopic dermatitis: three item severity score as a rough system for daily practice and as a pre-screening tool for studies. Acta Deml Venereol 1999;79(5):356-9.
22. Jenerowicz, D, Czarnecka-Operacz, M, Silny, W, Peripheral blood eosinophilia in atopic dermatitis. Acta Dermatovenerol Alp Panonica Adriat 2007;16(2):47-52.
23. Oranje, AP, Stalder, JF, Taieb, A et al. Scoring of atopic dermatitis by SCORAD using a training atlas by investigators from different disciplines. Pediatr Allergy Immunol 1997;8:28-4.
24. Sprikkelman, AB, Tupker, RA, Burgerhof, H et al. Severity scoring of atopic dermatitis: a comparison of three scoring systems. Allergy 1997;52:944-9.

## Claims

1. A composition comprising a mixture of plant extracts, wherein the mixture comprises:
an extract of *Althaea officinalis,* an extract of *Conium maculatum,* an extract of *Ervum lens,* an extract of *Phytolacca decandra,* an extract of *Populus alba,* an extract of *Populus tremuloides,* an extract of *Sambucus nigra,* an extract of *Hamamelis virginiana,* an extract of *Pimpinella saxifraga,* an extract of *Rhus toxicodendron,* an extract of *Vincetoxicum officinale,* an extract of *Achillea millefolium,* an extract of *Avena sativa,* an extract of *Sanguinaria canadensis,* an extract of *Berberis vulgaris,* an extract of *Cochlearia officinalis,* an extract of *Hydrastis canadensis,* an extract of *Matricaria chamomilla,* an extract of *Nasturtium officinale,* an extract of *Scrophularia nodosa,* an extract of *Smilax medica,* an extract of *Tussilago farfara,* and an extract of *Veronica officinalis.*

2. The composition of claim 1, wherein:
the extract of *Althaea officinalis* is present from about 0.01% to about 15%,
the extract of *Conium maculatum* is present from about 0.01% to about 30%,
the extract of *Ervum lens* is present from about 0.01% to about 17%,
the extract of *Phytolacca decandra* is present from about 0.01% to about 17%,
the extract of *Populus alba* is present from about 0.01% to about 17%,
the extract of *Populus tremuloides* is present from about 0.01% to about 17%,
the extract of *Sambucus nigra* is present from about 0.01% to about 15%,
the extract of *Hamamelis virginiana* is present from about 0.01% to about 25%,
the extract of *Pimpinella saxifraga* is present from about 0.01% to about 12%,
the extract of *Rhus toxicodendron* is present from about 0.01% to about 25%,
the extract of *Vincetoxicum officinale* is present from about 0.01% to about 12%,
the extract of *Achillea millefolium* is present from about 0.01% to about 15%,
the extract of *Avena sativa* is present from about 0.01% to about 25%,
the extract of *Sanguinaria canadensis* is present from about 0.01% to about 17%,
the extract of *Berberis vulgaris* is present from about 0.01% to about 17%,
the extract of *Cochlearia officinalis* is present from about 0.01% to about 17%,
the extract of *Hydrastis canadensis* is present from about 0.01% to about 17%,
the extract of *Matricaria chamomilla* is present from about 0.01% to about 17%,
the extract of *Nasturtium officinale* is present from about 0.01% to about 12%,
the extract of *Scrophularia nodosa* is present from about 0.01% to about 17%,
the extract of *Smilax medica* is present from about 0.01% to about 15%,
the extract of *Tussilago farfara* is present from about 0.01% to about 12%, and
the extract of *Veronica officinalis* is present from about 0.01% to about 12%.

3. The composition of claim 1, wherein:
the extract of *Althaea officinalis* is present from about 0.01% to about 10%,
the extract of *Conium maculatum* is present from about 0.01% to about 25%,
the extract of *Ervum lens* is present from about 0.01% to about 15%,
the extract of *Phytolacca decandra* is present from about 0.01% to about 15%,
the extract of *Populus alba* is present from about 0.01% to about 15%,
the extract of *Populus tremuloides* is present from about 0.01% to about 15%,
the extract of *Sambucus nigra* is present from about 0.01% to about 10%,
the extract of *Hamamelis virginiana* is present from about 0.01% to about 20%,
the extract of *Pimpinella saxifraga* is present from about 0.01% to about 10%,
the extract of *Rhus toxicodendron* is present from about 0.01% to about 20%,
the extract of *Vincetoxicum officinale* is present from about 0.01% to about 10%,
the extract of *Achillea millefolium* is present from about 0.01% to about 10%,
the extract of *Avena sativa* is present from about 0.01% to about 20%,
the extract of *Sanguinaria canadensis* is present from about 0.01% to about 15%,
the extract of *Berberis vulgaris* is present from about 0.01% to about 15%,
the extract of *Cochlearia officinalis* is present from about 0.01% to about 15%,
the extract of *Hydrastis canadensis* is present from about 0.01% to about 15%,
the extract of *Matricaria chamomilla* is present from about 0.01% to about 15%,
the extract of *Nasturtium officinale* is present from about 0.01% to about 10%,
the extract of *Scrophularia nodosa* is present from about 0.01% to about 15%,
the extract of *Smilax medica* is present from about 0.01% to about 10%,
the extract of *Tussilago farfara* is present from about 0.01% to about 10%, and
the extract of *Veronica officinalis* is present from about 0.01% to about 10%.

4. The composition of claim 1, wherein:
the extract of *Althaea officinalis* is present from about 0.01% to about 8%,
the extract of *Conium maculatum* is present from about 0.01% to about 23%,
the extract of *Ervum lens* is present from about 0.01% to about 12%,
the extract of *Phytolacca decandra* is present from about 0.01% to about 12%,
the extract of *Populus alba* is present from about 0.01% to about 12%,
the extract of *Populus tremuloides* is present from about 0.01% to about 12%,
the extract of *Sambucus nigra* is present from about 0.01% to about 8%,
the extract of *Hamamelis virginiana* is present from about 0.01% to about 17%,
the extract of *Pimpinella saxifraga* is present from about 0.01% to about 8%,
the extract of *Rhus toxicodendron* is present from about 0.01% to about 17%,
the extract of *Vincetoxicum officinale* is present from about 0.01% to about 8%,
the extract of *Achillea millefolium* is present from about 0.01% to about 8%,
the extract of *Avena sativa* is present from about 0.01% to about 17%,
the extract of *Sanguinaria canadensis* is present from about 0.01% to about 12%,
the extract of *Berberis vulgaris* is present from about 0.01% to about 12%,
the extract of *Cochlearia officinalis* is present from about 0.01% to about 12%,
the extract of *Hydrastis canadensis* is present from about 0.01% to about 12%,
the extract of *Matricaria chamomilla* is present from about 0.01% to about 12%,
the extract of *Nasturtium officinale* is present from about 0.01% to about 8%,
the extract of *Scrophularia nodosa* is present from about 0.01% to about 12%,
the extract of *Smilax medica* is present from about 0.01% to about 8%,
the extract of *Tussilago farfara* is present from about 0.01% to about 8%, and
the extract of *Veronica officinalis* is present from about 0.01% to about 8%.

5. The composition of claim 1, wherein:
the extract of *Althaea officinalis* is present from about 0.01% to about 7%,
the extract of *Ervum lens* is present from about 0.01% to about 10%,
the extract of *Phytolacca decandra* is present from about 0.01% to about 10%,
the extract of *Populus alba* is present from about 0.01% to about 10%,
the extract of *Populus tremuloides* is present from about 0.01% to about 10%,
the extract of *Sambucus nigra* is present from about 0.01% to about 7%,
the extract of *Hamamelis virginiana* is present from about 0.01% to about 15%,
the extract of *Pimpinella saxifraga* is present from about 0.01% to about 5%,
the extract of *Rhus toxicodendron* is present from about 0.01% to about 8.3%,
the extract of *Vincetoxicum officinale* is present from about 0.01% to about 5%,
the extract of *Achillea millefolium* is present from about 0.01% to about 7%,
the extract of *Avena sativa* is present from about 0.01% to about 15%,
the extract of *Sanguinaria canadensis* is present from about 0.01% to about 10%,
the extract of *Berberis vulgaris* is present from about 0.01% to about 10%,
the extract of *Cochlearia officinalis* is present from about 0.01% to about 10%,
the extract of *Hydrastis canadensis* is present from about 0.01% to about 10%,
the extract of *Matricaria chamomilla* is present from about 0.01% to about 10%,
the extract of *Nasturtium officinale* is present from about 0.01% to about 5%,
the extract of *Scrophularia nodosa* is present from about 0.01% to about 10%,
the extract of *Smilax medica* is present from about 0.01% to about 7%,
the extract of *Tussilago farfara* is present from about 0.01% to about 5%, and
the extract of *Veronica officinalis* is present from about 0.01% to about 5%.

6. The composition of claim 1, wherein:
the extract of *Althaea officinalis* is present from about 0.01% to about 5%,
the extract of *Sambucus nigra* is present from about 0.01% to about 6%,
the extract of *Achillea millefolium* is present from about 0.01% to about 6%, and
the extract of *Smilax medica* is present from about 0.01% to about 6.5%.

7. The composition of any one of claims 1 to 6, wherein said composition is formulated i) for oral administration, preferably in the form of a tablet, pill, dragee, capsule, liquid, gel, syrup, slurry, suspension, elixir or solution, or ii) for topical administration, preferably in the form of a solution, suspension, emulsion, syrup, gel, cream, lotion, or ointment.

8. A composition as defined in any of claims 1-7 for use in reducing symptoms associated with a skin condition in a subject.

9. The composition for use according to claim 8, wherein the skin condition is acne vulgaris, psoriasis, eczema, atopic dermatitis, pruritus, erythema, or excoriation.

10. A pharmaceutical composition comprising a composition of any of claims 1-9 and a pharmaceutically acceptable carrier.

## Patentansprüche

1. Zusammensetzung, umfassend eine Mischung von Pflanzenextrakten, wobei die Mischung umfasst:
ein Extrakt aus *Althaea officinalis,* ein Extrakt aus *Conium maculatum,* ein Extrakt aus *Ervum lens,* ein Extrakt aus *Phytolacca decandra,* ein Extrakt aus *Populus alba,* ein Extrakt aus *Populus tremuloides,* ein Extrakt aus *Sambucus nigra,* ein Extrakt aus *Hamamelis virginiana,* ein Extrakt aus *Pimpinella saxifraga,* ein Extrakt aus *Rhus toxicodendron,* ein Extrakt aus *Vincetoxicum officinale,* ein Extrakt aus *Achillea millefolium,* ein Extrakt aus *Avena sativa,* ein Extrakt aus *Sanguinaria canadensis,* ein Extrakt aus *Berberis vulgaris,* ein Extrakt aus *Cochlearia officinalis,* ein Extrakt aus *Hydrastis canadensis,* ein Extrakt aus *Matricaria chamomilla,* ein Extrakt aus *Nasturtium officinale,* ein Extrakt aus *Scrophularia nodosa,* ein Extrakt aus *Smilax medica,* ein Extrakt aus *Tussilago farfara* und ein Extrakt aus *Veronica officinalis.*

2. Zusammensetzung nach Anspruch 1, wobei
der Extrakt aus *Althaea officinalis* ist von etwa 0,01 % bis etwa 15 % enthalten,
der Extrakt aus *Conium maculatum* ist von etwa 0,01% bis etwa 30% enthalten,
der Extrakt aus der *Ervum-Linse* ist von etwa 0,01% bis etwa 17% enthalten,
der Extrakt aus *Phytolacca decandra* ist von etwa 0,01% bis etwa 17% enthalten,
der Extrakt von *Populus alba* ist von etwa 0,01% bis etwa 17% enthalten,
der Extrakt aus *Populus tremuloides* ist von etwa 0,01% bis etwa 17% enthalten,
der Extrakt aus *Sambucus nigra* ist von etwa 0,01% bis etwa 15% enthalten,
der Extrakt aus *Hamamelis virginiana* ist von etwa 0,01% bis etwa 25% enthalten,
der Extrakt aus *Pimpinella saxifraga* ist von etwa 0,01% bis etwa 12% enthalten,
der Extrakt aus *Rhus toxicodendron* ist von etwa 0,01% bis etwa 25% enthalten,
der Extrakt aus *Vincetoxicum officinale* ist von etwa 0,01 % bis etwa 12 % enthalten,
der Extrakt aus *Achillea millefolium* ist von etwa 0,01% bis etwa 15% enthalten,
der Extrakt von *Avena sativa* ist von etwa 0,01% bis etwa 25% enthalten,
der Extrakt aus *Sanguinaria canadensis* ist von etwa 0,01% bis etwa 17% enthalten,
der Extrakt aus *Berberis vulgaris* ist von etwa 0,01% bis etwa 17% enthalten,
der Extrakt aus *Cochlearia officinalis* ist von etwa 0,01 % bis etwa 17 % enthalten.
der Extrakt aus *Hydrastis canadensis* ist von etwa 0,01% bis etwa 17% enthalten,
der Extrakt aus *Matricaria chamomilla* ist von etwa 0,01% bis etwa 17% enthalten,
der Extrakt aus *Nasturtium officinale* ist von etwa 0,01 % bis etwa 12 % enthalten.
der Extrakt aus *Scrophularia nodosa* ist von etwa 0,01% bis etwa 17% enthalten,
der Extrakt von *Smilax medica* ist von etwa 0,01% bis etwa 15% enthalten,
der Extrakt aus *Tussilago farfara* ist von etwa 0,01 % bis etwa 12 % enthalten und
der Extrakt von *Veronica officinalis* ist von etwa 0,01% bis etwa 12% enthalten.

3. Zusammensetzung nach Anspruch 1, wobei
der Extrakt aus *Althaea officinalis* ist von etwa 0,01 % bis etwa 10 % enthalten.
der Extrakt aus *Conium maculatum* ist von etwa 0,01% bis etwa 25% enthalten,
der Extrakt aus der *Ervum-Linse* ist von etwa 0,01% bis etwa 15% enthalten,
der Extrakt aus *Phytolacca decandra* ist von etwa 0,01% bis etwa 15% enthalten,
der Extrakt von *Populus alba* ist von etwa 0,01% bis etwa 15% enthalten,
der Extrakt aus *Populus tremuloides* ist von etwa 0,01% bis etwa 15% enthalten,
der Extrakt aus *Sambucus nigra* ist von etwa 0,01% bis etwa 10% enthalten,
der Extrakt aus *Hamamelis virginiana* ist von etwa 0,01% bis etwa 20% enthalten,
der Extrakt aus *Pimpinella saxifraga* ist von etwa 0,01% bis etwa 10% enthalten,
der Extrakt aus *Rhus toxicodendron* ist von etwa 0,01% bis etwa 20% enthalten,
der Extrakt aus *Vincetoxicum officinale* ist von etwa 0,01 % bis etwa 10 % enthalten,
der Extrakt aus *Achillea millefolium* ist von etwa 0,01% bis etwa 10% enthalten,
der Extrakt von *Avena sativa* ist von etwa 0,01% bis etwa 20% enthalten,
der Extrakt aus *Sanguinaria canadensis* ist von etwa 0,01% bis etwa 15% enthalten,
der Extrakt aus *Berberis vulgaris* ist von etwa 0,01% bis etwa 15% enthalten,
der Extrakt aus *Cochlearia officinalis* ist zwischen etwa 0,01 % und etwa 15 % enthalten.
der Extrakt aus *Hydrastis canadensis* ist von etwa 0,01 % bis etwa 15 % enthalten,
der Extrakt aus *Matricaria chamomilla* ist von etwa 0,01% bis etwa 15% enthalten,
der Extrakt aus *Nasturtium officinale* ist von etwa 0,01 % bis etwa 10 % enthalten,
der Extrakt aus *Scrophularia nodosa* ist von etwa 0,01% bis etwa 15% enthalten,
der Extrakt von *Smilax medica* ist von etwa 0,01% bis etwa 10% enthalten,
der Extrakt aus *Tussilago Farfara* ist von etwa 0,01 % bis etwa 10 % enthalten und
der Extrakt von *Veronica officinalis* ist von etwa 0,01% bis etwa 10% enthalten.

4. Zusammensetzung nach Anspruch 1, wobei
der Extrakt aus *Althaea officinalis* ist von etwa 0,01 % bis etwa 8 % enthalten.
der Extrakt aus *Conium maculatum* ist von etwa 0,01% bis etwa 23% enthalten,
der Extrakt aus der *Ervum-Linse* ist von etwa 0,01% bis etwa 12% enthalten,
der Extrakt aus *Phytolacca decandra* ist von etwa 0,01% bis etwa 12% enthalten,
der Extrakt von *Populus alba* ist von etwa 0,01% bis etwa 12% enthalten,
der Extrakt von *Populus tremuloides* ist von etwa 0,01% bis etwa 12% enthalten,
der Extrakt aus *Sambucus nigra* ist von etwa 0,01% bis etwa 8% enthalten,
der Extrakt von *Hamamelis virginiana* ist von etwa 0,01% bis etwa 17% enthalten,
der Extrakt aus *Pimpinella saxifraga* ist von etwa 0,01% bis etwa 8% enthalten,
der Extrakt aus *Rhus toxicodendron* ist von etwa 0,01 % bis etwa 17 % enthalten,
der Extrakt aus *Vincetoxicum officinale* ist von etwa 0,01 % bis etwa 8 % enthalten.
der Extrakt aus *Achillea millefolium* ist von etwa 0,01% bis etwa 8% enthalten,
der Extrakt von *Avena sativa* ist von etwa 0,01% bis etwa 17% enthalten,
der Extrakt aus *Sanguinaria canadensis* ist von etwa 0,01% bis etwa 12% enthalten,
der Extrakt aus *Berberis vulgaris* ist von etwa 0,01% bis etwa 12% enthalten,
der Extrakt aus *Cochlearia officinalis* ist von etwa 0,01 % bis etwa 12 % enthalten.
der Extrakt aus *Hydrastis canadensis* ist von etwa 0,01% bis etwa 12% enthalten,
der Extrakt aus *Matricaria chamomilla* ist von etwa 0,01% bis etwa 12% enthalten,
der Extrakt aus *Nasturtium officinale* ist von etwa 0,01% bis etwa 8% enthalten,
der Extrakt aus *Scrophularia nodosa* ist von etwa 0,01% bis etwa 12% enthalten,
der Extrakt von *Smilax medica* ist von etwa 0,01% bis etwa 8% enthalten,
der Extrakt aus *Tussilago farfara* ist von etwa 0,01 % bis etwa 8 % enthalten
der Extrakt von *Veronica officinalis* ist von etwa 0,01% bis etwa 8% enthalten.

5. Zusammensetzung nach Anspruch 1, wobei
der Extrakt aus *Althaea officinalis* ist von etwa 0,01 % bis etwa 7 % enthalten.
der Extrakt aus *der Ervum-Linse* ist von etwa 0,01% bis etwa 10% enthalten,
der Extrakt aus *Phytolacca decandra* ist von etwa 0,01% bis etwa 10% enthalten,
der Extrakt von *Populus alba* ist von etwa 0,01% bis etwa 10% enthalten,
der Extrakt aus *Populus tremuloides* ist von etwa 0,01% bis etwa 10% enthalten,
der Extrakt aus *Sambucus nigra* ist von etwa 0,01 % bis etwa 7 % enthalten.
der Extrakt aus *Hamamelis virginiana* ist von etwa 0,01% bis etwa 15% enthalten,
der Extrakt aus *Pimpinella saxifraga* ist von etwa 0,01% bis etwa 5% enthalten,
der Extrakt aus *Rhus toxicodendron* ist von etwa 0,01 % bis etwa 8,3 % enthalten,
der Extrakt aus *Vincetoxicum officinale* ist von etwa 0,01 % bis etwa 5 % enthalten.
der Extrakt aus *Achillea millefolium* ist von etwa 0,01% bis etwa 7% enthalten,
der Extrakt von *Avena sativa* ist von etwa 0,01% bis etwa 15% enthalten,
der Extrakt aus *Sanguinaria canadensis* ist von etwa 0,01% bis etwa 10% enthalten,
der Extrakt aus *Berberis vulgaris* ist von etwa 0,01% bis etwa 10% enthalten,
der Extrakt aus *Cochlearia officinalis* ist von etwa 0,01 % bis etwa 10 % enthalten.
der Extrakt aus *Hydrastis canadensis* ist von etwa 0,01% bis etwa 10% vorhanden,
der Extrakt aus *Matricaria chamomilla* ist von etwa 0,01% bis etwa 10% enthalten,
der Extrakt aus *Nasturtium officinale* ist von etwa 0,01 % bis etwa 5 % enthalten.
der Extrakt aus *Scrophularia nodosa* ist von etwa 0,01% bis etwa 10% enthalten,
der Extrakt von *Smilax medica* ist von etwa 0,01% bis etwa 7% enthalten,
der Extrakt aus *Tussilago farfara* liegt bei etwa 0,01 % bis etwa 5 % und
der Extrakt von *Veronica officinalis* ist von etwa 0,01% bis etwa 5% enthalten.

6. Zusammensetzung nach Anspruch 1, wobei
der Extrakt aus *Althaea officinalis* ist von etwa 0,01 % bis etwa 5 % enthalten.
der Extrakt aus *Sambucus nigra* ist von etwa 0,01% bis etwa 6% enthalten,
der Extrakt aus *Achillea millefolium* liegt bei etwa 0,01 % bis etwa 6 % und
der Extrakt von *Smilax medica* ist von etwa 0,01% bis etwa 6,5% enthalten.

7. Zusammensetzung eines der Ansprüche 1 bis 6, wobei die Zusammensetzung i) zur oralen Verabreichung, vorzugsweise in Form einer Tablette, Pille, Dragee, Kapsel, Flüssigkeit, Gel Sirup, Aufschlämmung, Suspension, Elixier oder Lösung, oder ii) zur topischen Verabreichung, vorzugsweise in Form einer Lösung, Suspension, Emulsion, Sirup, Gel Creme, Lotion oder Salbe formuliert ist.

8. Zusammensetzung nach einem der Ansprüche 1 bis 7 zur Verwendung zur Verringerung von Symptomen, die mit einer Hauterkrankung bei einem Subjekt verbunden sind.

9. Zusammensetzung zur Verwendung nach Anspruch 8, wobei der Hautzustand Akne vulgaris, Psoriasis, Ekzem, atopische Dermatitis, Juckreiz, Erythem oder Exkoriation ist.

10. Pharmazeutische Zusammensetzung, umfassend eine Zusammensetzung eines der Ansprüche 1 bis 9 und einen pharmazeutisch akzeptablen Träger.

## Revendications

1. Composition comprenant un mélange d'extraits de plantes, dans laquelle le mélange comprend :
un extrait d'*Althaea officinalis,* un extrait de *Conium maculatum,* un extrait d'*Ervum lens,* un extrait de *Phytolacca decandra,* un extrait de *Populus alba,* un extrait de *Populus tremuloides,* un extrait de *Sambucus nigra,* un extrait d'*Hamamelis virginiana,* un extrait de *Pimpinella saxifraga,* un extrait de *Rhus toxicodendron,* un extrait de *Vincetoxicum officinale,* un extrait *d'Achillea millefolium,* un extrait d'*Avena sativa,* un extrait de *Sanguinaria canadensis,* un extrait de *Berberis vulgaris,* un extrait de *Cochlearia officinalis,* un extrait d'*Hydrastis canadensis,* un extrait de *Matricaria chamomilla,* un extrait de *Nasturtium officinale,* un extrait de *Scrophularia nodosa,* un extrait de *Smilax medica,* un extrait de *Tussilago farfara* et un extrait de *Veronica officinalis.*

2. Composition selon la revendication 1, dans laquelle :
l'extrait *d' Althaea officinalis* est présent d'environ 0,01 % à environ 15 %,
l'extrait de *Conium maculatum* est présent d'environ 0,01 % à environ 30 %,
l'extrait de lentille d' *Ervum* est présent d'environ 0,01 % à environ 17 %,
l'extrait de *Phytolacca decandra* est présent d'environ 0,01 % à environ 17 %,
l'extrait de *Populus alba* est présent d'environ 0,01 % à environ 17 %,
l'extrait de *Populus tremuloides* est présent d'environ 0,01 % à environ 17 %,
l'extrait de *Sambucus nigra* est présent d'environ 0,01 % à environ 15 %,
l'extrait *d' Hamamelis virginiana* est présent d'environ 0,01 % à environ 25 %,
l'extrait de *Pimpinella saxifraga* est présent d'environ 0,01 % à environ 12 %,
l'extrait de *Rhus toxicodendron* est présent d'environ 0,01 % à environ 25 %,
l'extrait de *Vincetoxicum officinale* est présent d'environ 0,01 % à environ 12 %,
l'extrait d' *Achillea millefolium* est présent d'environ 0,01 % à environ 15 %,
l'extrait d' *Avena sativa* est présent d'environ 0,01 % à environ 25 %,
l'extrait de *Sanguinaria canadensis* est présent d'environ 0,01 % à environ 17 %,
l'extrait de *Berberis vulgaris* est présent d'environ 0,01 % à environ 17 %,
l'extrait de *Cochlearia officinalis* est présent d'environ 0,01 % à environ 17 %,
l'extrait d' *Hydrastis canadensis* est présent d'environ 0,01 % à environ 17 %,
l'extrait de *Matricaria chamomilla* est présent d'environ 0,01 % à environ 17 %,
l'extrait de *Nasturtium officinale* est présent d'environ 0,01 % à environ 12 %,
l'extrait de *Scrophularia nodosa* est présent d'environ 0,01 % à environ 17 %,
l'extrait de *Smilax medica* est présent d'environ 0,01 % à environ 15 %,
l'extrait de *Tussilago farfara* est présent d'environ 0,01 % à environ 12 %, et
l'extrait de *Veronica officinalis* est présent d'environ 0,01 % à environ 12 %.

3. Composition selon la revendication 1, dans laquelle :
l'extrait d' *Althaea officinalis* est présent d'environ 0,01 % à environ 10 %,
l'extrait de *Conium maculatum* est présent d'environ 0,01 % à environ 25 %,
l'extrait de *lentille Ervum* est présent d'environ 0,01 % à environ 15 %,
l'extrait de *Phytolacca decandra* est présent d'environ 0,01 % à environ 15 %,
l'extrait de *Populus alba* est présent d'environ 0,01 % à environ 15 %,
l'extrait de *Populus tremuloides* est présent d'environ 0,01 % à environ 15 %,
l'extrait de *Sambucus nigra* est présent d'environ 0,01 % à environ 10 %,
l'extrait d' *Hamamelis virginiana* est présent d'environ 0,01 % à environ 20 %,
l'extrait de *Pimpinella saxifraga* est présent d'environ 0,01 % à environ 10 %,
l'extrait de *Rhus toxicodendron* est présent d'environ 0,01 % à environ 20 %,
l'extrait de *Vincetoxicum officinale* est présent d'environ 0,01 % à environ 10 %,
l'extrait d' *Achillea millefolium* est présent d'environ 0,01 % à environ 10 %,
l'extrait d' *Avena sativa* est présent d'environ 0,01 % à environ 20 %,
l'extrait de *Sanguinaria canadensis* est présent d'environ 0,01 % à environ 15 %,
l'extrait de *Berberis vulgaris* est présent d'environ 0,01 % à environ 15 %,
l'extrait de *Cochlearia officinalis* est présent d'environ 0,01 % à environ 15 %,
l'extrait d' *Hydrastis canadensis* est présent d'environ 0,01 % à environ 15 %,
l'extrait de *Matricaria chamomilla* est présent d'environ 0,01 % à environ 15 %,
l'extrait de *Nasturtium officinale* est présent d'environ 0,01 % à environ 10 %,
l'extrait de *Scrophularia nodosa* est présent d'environ 0,01 % à environ 15 %,
l'extrait de *Smilax medica* est présent d'environ 0,01 % à environ 10 %,
l'extrait de *Tussilago farfara* est présent d'environ 0,01 % à environ 10 %, et
l'extrait de *Veronica officinalis* est présent d'environ 0,01 % à environ 10 %.

4. Composition selon la revendication 1, dans laquelle :
l'extrait d' *Althaea officinalis* est présent d'environ 0,01 % à environ 8 %,
l'extrait de *Conium maculatum* est présent d'environ 0,01 % à environ 23 %,
l'extrait de *lentille Ervum* est présent d'environ 0,01 % à environ 12 %,
l'extrait de *Phytolacca decandra* est présent d'environ 0,01 % à environ 12 %,
l'extrait de *Populus alba* est présent d'environ 0,01 % à environ 12 %,
l'extrait de *Populus tremuloides* est présent d'environ 0,01 % à environ 12 %,
l'extrait de *Sambucus nigra* est présent d'environ 0,01 % à environ 8 %,
l'extrait d' *Hamamelis virginiana* est présent d'environ 0,01 % à environ 17 %,
l'extrait de *Pimpinella saxifraga* est présent d'environ 0,01 % à environ 8 %,
l'extrait de *Rhus toxicodendron* est présent d'environ 0,01 % à environ 17 %,
l'extrait de *Vincetoxicum officinale* est présent d'environ 0,01 % à environ 8 %,
l'extrait d' *Achillea millefolium* est présent d'environ 0,01 % à environ 8 %,
l'extrait d' *Avena sativa* est présent d'environ 0,01 % à environ 17 %,
l'extrait de *Sanguinaria canadensis* est présent d'environ 0,01 % à environ 12 %,
l'extrait de *Berberis vulgaris* est présent d'environ 0,01 % à environ 12 %,
l'extrait de *Cochlearia officinalis* est présent d'environ 0,01 % à environ 12 %,
l'extrait d' *Hydrastis canadensis* est présent d'environ 0,01 % à environ 12 %,
l'extrait de *Matricaria chamomilla* est présent d'environ 0,01 % à environ 12 %,
l'extrait de *Nasturtium officinale* est présent d'environ 0,01 % à environ 8 %,
l'extrait de *Scrophularia nodosa* est présent d'environ 0,01 % à environ 12 %,
l'extrait de *Smilax medica* est présent d'environ 0,01 % à environ 8 %,
l'extrait de *Tussilago farfara* est présent d'environ 0,01 % à environ 8 %, et
l'extrait de *Veronica officinalis* est présent d'environ 0,01 % à environ 8 %.

5. Composition selon la revendication 1, dans laquelle :
l'extrait d' *Althaea officinalis* est présent d'environ 0,01 % à environ 7 %,
l'extrait de *lentille Ervum* est présent d'environ 0,01 % à environ 10 %,
l'extrait de *Phytolacca decandra* est présent d'environ 0,01 % à environ 10 %,
l'extrait de *Populus alba* est présent d'environ 0,01 % à environ 10 %,
l'extrait de *Populus tremuloides* est présent d'environ 0,01 % à environ 10 %,
l'extrait de *Sambucus nigra* est présent d'environ 0,01 % à environ 7 %,
l'extrait d' *Hamamelis virginiana* est présent d'environ 0,01 % à environ 15 %,
l'extrait de *Pimpinella saxifraga* est présent d'environ 0,01 % à environ 5 %,
l'extrait de *Rhus toxicodendron* est présent d'environ 0,01 % à environ 8,3 %,
l'extrait de *Vincetoxicum officinale* est présent d'environ 0,01 % à environ 5 %,
l'extrait d' *Achillea millefolium* est présent d'environ 0,01 % à environ 7 %,
l'extrait d' *Avena sativa* est présent d'environ 0,01 % à environ 15 %,
l'extrait de *Sanguinaria canadensis* est présent d'environ 0,01 % à environ 10 %,
l'extrait de *Berberis vulgaris* est présent d'environ 0,01 % à environ 10 %,
l'extrait de *Cochlearia officinalis* est présent d'environ 0,01 % à environ 10 %,
l'extrait d' *Hydrastis canadensis* est présent d'environ 0,01 % à environ 10 %,
l'extrait de *Matricaria chamomilla* est présent d'environ 0,01 % à environ 10 %,
l'extrait de *Nasturtium officinale* est présent d'environ 0,01 % à environ 5 %,
l'extrait de *Scrophularia nodosa* est présent d'environ 0,01 % à environ 10 %,
l'extrait de *Smilax medica* est présent d'environ 0,01 % à environ 7 %,
l'extrait de *Tussilago farfara* est présent d'environ 0,01 % à environ 5 %, et
l'extrait de *Veronica officinalis* est présent d'environ 0,01 % à environ 5 %.

6. Composition selon la revendication 1, dans laquelle :
l'extrait d' *Althaea officinalis* est présent d'environ 0,01 % à environ 5 %,
l'extrait de *Sambucus nigra* est présent d'environ 0,01 % à environ 6 %,
l'extrait d' *Achillea millefolium* est présent d'environ 0,01 % à environ 6 %, et
l'extrait de *Smilax medica* est présent d'environ 0,01 % à environ 6,5 %.

7. Composition selon l'une quelconque des revendications 1 à 6, dans laquelle ladite composition est formulée i) pour une administration orale, de préférence sous la forme d'un comprimé, d'une pilule, d'une dragée, d'une capsule, d'un liquide, d'un gel, d'un sirop, d'une bouillie, d'une suspension, d'un élixir ou d'une solution, ou ii) pour une administration topique, de préférence sous la forme d'une solution, d'une suspension, d'une émulsion, d'un sirop, d'un gel, d'une crème, d'une lotion ou d'une pommade.

8. Composition telle que définie dans l'une quelconque des revendications 1 à 7, destinée à être utilisée pour réduire les symptômes associés à une affection cutanée chez un sujet.

9. Composition à utiliser selon la revendication 8, dans laquelle l'affection cutanée est l'acné vulgaire, le psoriasis, l'eczéma, la dermatite atopique, le prurit, l'érythème ou l'excoriation.

10. Composition pharmaceutique comprenant une composition selon l'une quelconque des revendications 1 à 9 et un support pharmaceutiquement acceptable.
